# EUROPEAN PATENT APPLICATION

(11) **EP 4 825 408 A2**
(43) Date of publication of application: **21.10.2026**
(21) Application number: 26203669.2
(22) Date of filing: 07.11.2014
(51) Int. Cl.: A61M 5/00

(54) **APPARATUS FOR ACCESSING AND SEALING BODILY VESSELS AND CAVITIES**

(30) Priority: 11.11.2013 US 201361902742 P; 09.04.2014 US 201461977478 P; 30.05.2014 US 201462005355 P; 03.06.2014 US 201462007339 P; 27.10.2014 US 201414524980; 27.10.2014 US 201414525012; 27.10.2014 US 201414525043
(62) Document number(s) of the earlier application(s) in accordance with Art. 76 EPC: 25157384.6 / 4 529 935; 14860764.1 / 3 068 478
(71) Applicant: CrossBay Medical, Inc., San Diego, CA 92128 (US)
(72) Inventor: BACICH, Steven R., Half Moon Bay, CA, 94019 (US); VIDYARTHI, Piush, San Rafael, CA, 94901 (US); YUREK, Matthew, San Diego, CA, 92129 (US); GREELIS, Jack, Carlsbad, CA, 92009 (US)
(74) Representative: Lorenz Seidler Gossel Part. mbB

(57) **Abstract**

Everting balloon systems and methods for using the same are disclosed herein. The systems can be configured to access and dilate body lumen and cavities. For example, the systems can be used to dilate the cervix and access the uterine cavity. The systems can also be used to occlude the cervix. The systems can also be used to occlude the urethra.

## Description

### CROSS-REFERENCE TO RELATED APPLICATIONS

This application is a continuation of U.S. Application Nos. 14/524,980 filed October 27, 2014; 14/525,012 filed October 27, 2014; and 14/525,043 filed October 27, 2014, each of which claim priority to U.S. Provisional Application Nos. 61/902,742, filed November 11, 2013; 61/977,478, filed April 9, 2014; 62/005,355, filed May 30, 2014; and 62/007,339, filed June 3, 2014, all of which are incorporated by reference herein in their entireties.

### BACKGROUND

For physicians and medical professionals, accessing systems for vessels and bodily cavities in patients have typically used various guidewire and catheter technologies. In some cases, the process requires the insertion of a series of mandrels or wires to increase the lumen diameter for the eventual passage of a larger bore instrument within the vessel. This technique can be referred to as "Dottering" or in the case of accessing the cervical canal and uterus, physicians will use a series of increasing diameter mandrels known as Hegar dilators. In the techniques described above, the methods involved pushing an object, mandrel, or device through the vessel to gain access to a desired region in the body. The result of pushing an object, mandrel, or device creates shear forces on the lumen wall. In some cases the shear forces can result in trauma, pain for the patient, or perforation.

In contrast, another access technology that has been used in prior art is referred to as an everting catheter. Everting catheters utilize a traversing action in which a balloon is inverted and with the influence of hydraulic pressure created by a compressible or incompressible fluid or media, rolls inside out or everts with a propulsion force through the vessel. Everting balloons have been referred to as rolling or outrolling balloons, evaginating membranes, toposcopic catheters, or linear everting catheters such as those in U.S. Patent Nos. 5,364,345; 5,372,247; 5,458,573; 5,472,419; 5,630,797; 5,902,286; 5,993,427; 6,039,721; 3,421,509; and 3,911,927; all of which are incorporated herein by reference in their entireties. These are categorized as everting balloons and are for traversing vessels, cavities, tubes, or ducts in a frictionless manner. In other words, an everting balloon can traverse a tube without imparting any shear forces on the wall being traversed. Because of this action and lack of shear forces, resultant trauma can be reduced and the risk of perforation reduced. In addition as a result of the mechanism of travel through a vessel, material and substances in the proximal portion of the tube or vessel are not pushed or advanced forward to a more distal portion of the tube or vessel.

In addition, as the everting catheter deploys inside out, uncontaminated or untouched balloon material is placed inside the vessel wall. In the inverted or undeployed state, the balloon is housed inside the catheter body and cannot come into contact with the patient or physician. As the balloon is pressurized and everted, the balloon material rolls inside out without contacting any element outside of the vessel. Another advantage of an everting balloon catheter is that the method of access is more comfortable for the patient since the hydraulic forces "pull" the balloon membrane through the vessel or duct as opposed to a standard catheter that needs to be "pushed" into and through the vessel or duct.

The method typically used by physicians for accessing the cervical canal in women requires the use of multiple instruments of increasing diameter. The physician will use a small uterine sound or small diameter probe or Hegar device for gaining initial entry into the uterus via the cervix. Ever increasing sizes of Hegars are used to stretch the cervical muscles until the desired internal diameter is achieved for the insertion of a secondary instrument such as an endoscope or other device. This process can be particularly difficult in some nulliparous, post-menopausal women with very small diameter cervical canals. A cervix could be difficult to traverse as a result of prior surgery, underlying stenosis, or other anatomical configuration or tortuosity that makes the passage of instruments or Hegar dilators difficult.

There are some cervical dilators that provide radial expansion to open the cervical canal to a greater internal diameter without the insertion of multiple instruments. All of these devices are predicated on first crossing or traversing the cervical canal prior to the step of radial expansion. Once traversed through the cervical canal, these devices use either mechanical means or the expansion of a balloon dilation member that is concentric on the exterior of the dilator probe. If the cervical canal is particularly tight or narrow, a small diameter probe or mandrel may be required to first cross the cervix and access the uterine cavity. As mandrels or instruments get smaller in diameter, the likelihood of perforation or a false passage increases. In any case, these cervical dilators require passage or crossing by the initial probe prior to any further radial expansion being performed.

Everting catheters have been described as dilatation catheters. Representative examples of dilating everting catheters include U.S. Patent Nos. 5,364,345 and 4,863,440, both of which are incorporated by reference herein in their entireties.

Everting catheters have also been described with additional elements such as a handle for controlling instruments within an everting catheter. A representative example is U.S. Patent No. 5,346,498 which is incorporated by reference herein in its entirety. Everting balloon catheters can be constructed with an inner catheter with an internal lumen or through-lumen (or thru-lumen). The through-lumen can be used for the passage of instruments, media, materials, therapeutic agents, endoscope, guidewires, or other instruments. Representative samples of everting catheters with through-lumens are in US Patent No. 5,374,247 and 5,458,573. In addition, everting catheters have been described with waists or a narrowing of the balloon diameter, such as in U.S. Patent No. 5,074,845, which is incorporated by reference herein in its entirety.

Furthermore, infertility is a condition that affects 1 out of 8 couples in the US. One of the early treatments in the infertility regime is insemination. Intrauterine insemination or IUI is a very common procedure since it is in the early work up of an infertile couple. Most assisted reproductive clinics perform at least 3 IUI cycles before trying more expensive treatment options such as IVF. IUI cycles may be coupled with drugs to stimulate greater ovulation to improve the chances or likelihood of pregnancy. Sperm that is collected from the male is typically washed and prepared prior to an insemination through a catheter with the goal of providing a greater volume or amount of viable sperm in the uterine cavity and into the reproductive tract of the female.

As part of increasing the odds of getting pregnant, physicians have been using a variety of means of increasing both the amount of sperm and the duration sperm could be in the uterine cavity. To keep sperm from exiting the cervical canal after insemination, physicians have used rubber and silicone based cervical caps that are designed to fit over the exocervix as a sealing member. In practice, a physician will either inseminate the uterine cavity or fill the cervical cap with sperm and then fit over the cervix as a seal. The security of the cervical cap is not reliable and due to its size and bulk, not as comfortable for the patient. Typically a cervical cap will only be used by the patient within the physician's office for a predetermined amount of time. The overall objective of the cervical cap is to keep sperm in the uterine cavity for as long as possible without being expelled or spilling from the cervix due to gravity or contractions of the uterus. Ideally the sperm will migrate upward through the fallopian tubes to the fimbria where conception can occur if an oocyte from an ovary is present. Many clinical investigations have reported that, once through the fallopian tubes, sperm can travel throughout the peritoneal cavity and stay viable for many hours in situ. Equally, sperm can stay viable in a controlled environment or an incubator for many hours.

Another methodology for keeping sperm in the uterine cavity is done by use of a pump connected to a catheter within the uterine cavity. A pump that can be worn by the women or keep bedside is filled with sperm and is pumped through the catheter at a rate pre-set by the physician. For instance, the pump can be set to run for 2 to 6 hours at which sperm is pumped through the catheter and into the uterine cavity. The pump can be worn by the woman outside of the physician's office but patient mobility is limited due to keeping the integrity of the conduit and pump.

Also, when delivering the reproductive material, such as an embryo, into the uterine cavity, vacuum effect can unintentionally remove the reproductive material from the uterine cavity. In existing systems, when the transfer catheter is retracted from a second outer or guiding catheter (e.g., the "inner" catheter), the retraction produces vacuum pressure within the uterine cavity. This vacuum pressure is created in the uterine cavity by the removal and backward movement of the transfer catheter within the inner catheter. After the embryo transfer is completed, an embryologist may inspect the transfer catheter to verify that the embryos or reproductive material was indeed deposited in the uterus and not pulled back into the transfer catheter because of the vacuum effect. The same procedure may be done for the outer catheter once this catheter is removed.

Further, incontinence is a prevalent clinical and social issue that primarily affects women. Various publications estimate the percentage of women with urinary incontinence from 15 to 30% in the over 60 age group. The amount spent treating incontinence ranges from $3 to 5B annually in the US. Urinary Incontinence is one of the 10 most common chronic conditions in the US with over 15M women diagnosed in the US alone. Most feel that this number is under-reported. The choice of therapeutic options has limited reimbursement and many of the techniques lack consistent data. As an example, invasive approaches in women include suspensions of the bladder neck, collagen injections, and RF remodeling. Incontinence is a multi-factorial disease condition and patient satisfaction with many invasive techniques is poor. For patients, surgery is the last preferred resort. Noninvasive techniques include biofeedback and Kegel exercisers. These techniques improve the muscle tone in the patient's pelvic floor but they rely on patient compliance to be effective. Less invasive options involve mechanical pressure devices such as pessaries, urethral catheters or inserts, and patches over the urethral opening. Most incontinence money is expended on purchase of absorbent products. Due to the social stigma, many patients are reluctant to seek advice and many patients decline surgical and less invasive options. Consequently, male and female incontinence patients rely on sanitary pads and the restrictions that the presence of bulky, wet pads imposes on their daily lives.

In some patients, a urethral insertable device for sealing is the preferred method for managing their incontinence. These inserts are designed for single use and are self-applied. Typically they rely on the measurements of urethral length for insertion and balloon inflation.

Urethral inserts are plugs that typically incorporate a balloon mechanism to create a seal in the urethra or bladder. For voiding, the balloon is deflated. These devices have been described previously in the art including US Patent No. 5,090,424; 5,483,976; 5,724,994; 5,752,525; and 5,769,091 all attributed to Simon et al. Additional art is found in U.S. Patent No. 5,806,527; 6,449,060; 6,926,708; EP 0900058 B1, and EP 1365713 B1. U.S. Patent No 5,927,282 to Lenker et al describes an adhesive patch for covering and sealing the urethral opening and sealing the external opening to the urethra.

Further art includes U.S. Patent No. 5,662,582 which describes an everting urethral plug with an invaginating balloon mechanism. Everting balloons describe an action in which a balloon is inverted and, with the influence of hydraulic pressure created by a compressible or incompressible fluid or media, rolls inside out or everts with that propulsion force. Everting balloons have been referred to as rolling or outrolling balloons, evaginating membranes, toposcopic catheters, or linear everting balloons. These are all categorized as everting balloons due to their property of traversing vessels, cavities, tubes, or ducts in a substantially frictionless manner. Everting balloons can traverse a tube without imparting any significant shear forces on the wall being traversed. Because of this action and lack of shear forces, material and substances in the proximal portion of the tube or vessel are pushed or advanced forward to a more distal portion of the tube or vessel. For example for urethral everting balloons, potentially infectious substances from the vagina, urethral openings, or the hands of the patient, are not in contact with the everted balloon that resides in the urethra. In contrast, urethral plugs and inserts in use currently have a propensity of urinary tract infections which may be a result of the lack of cleanliness of the device that resides in the urethra or bladder.

This methodology would be more widely adopted if the inserts were more comfortable while wearing, had less of a foreign body sensation, and were easier to use or insert. More significantly, many patients who are insert users complain of higher incidence of urethral infection and soreness at their urethra and groin region, especially with increased activity, which is the ideal time the user would want the insert to operate properly.

For a patient, being able to insert a device within their own urethra is not easy and the act itself is unnatural for the patient. Training by a nurse or physician is required for a patient to master the technique. Since the inserts used today require pushing a device through the urethra, the urethral passageway could become sore or tender after repeated insertions.

Current urethra inserts are typically sized to match the length of the patient's urethra, not as a "one size fits all." Current inserts need to create a balloon seal at the bladder coupled with a compressive force at the urethral opening. Thus the insert length is calculated by sounding the length of the urethra by a probe or via ultrasound measurement. The calculation is designed to hold the balloon taut against the opening of the bladder and the compressive force of the external portion of the insert of the opening of the urethra. Hence it is understandable that the presence of a relatively stiff device that is keeping the opening of the bladder tight against the urethral opening can create an uncomfortable feeling for the patient, especially a patient that is active and mobile.

Furthermore, urinary protection and voiding could occur 3 to 7 times a day, a high cost device would not be acceptable to most patients or would limit the device usage to only the most socially challenging events.

### SUMMARY OF THE INVENTION

An everting balloon system is disclosed. The everting balloon system can be used for insemination, urinary incontinence, dilation of a body lumen, for access and sealing within a body cavity, or combinations thereof. The system can have automatic disengagement. The system can have a handle for insertion. The system can have a motorized air pump. The system can have inner and outer catheters that can automatically disengage upon everting.

The everting balloon system can have deflation and removal mechanisms of a device that can be worn by a mobile patient in a body lumen (e.g., cervical canal or urethra) and be deflated and removed by the patient.

The everting balloon system can have an intubating base with a locking balloon that can activate upon pressurization. The system can be a compact, low profile unit used in vivo. The system can be single use and disposable. The system can be non-irritation and non-infection causing.

The everting balloon system can be used for cervical access and dilation. The everting balloon system can have a system handle mechanism that can enable a one-handed operating technique by the user. The one-handed operating technique can include advancement and pressurization of the everting balloon membrane within the control of the user with one hand.

The everting balloon system can be used for insemination and can seal the cervix for a duration of time for the deposition of sperm and to allow for mobility for the patient. The everting balloon system can have a decoupling mechanism configured to decouple the outer catheter and inner catheter while maintaining hydraulic pressure in an everting balloon. The system can deflate and removal the everting balloon concurrently.

The system can be used to place or deliver fallopian tube inserts (i.e., intratubal inserts, such as the Essure device from Bayer Corporation) in fallopian tubes. The system can access the intramural and isthmic portions of the fallopian tube. All or part of the everting catheter system can be loaded into a hysteroscope and placed with direct endoscopic visualization.

The everting catheter system can be a selective fallopian tube catheter with a curved distal end section and angled ball tip. This configuration can be performed by ultrasound or radiographic visualization.

One or more fallopian tube occluding devices (e.g., the Essure device) can be loaded into the everting balloon system, for example, in the through lumen of the inner catheter. Once fully everted and placed into the fallopian tube, the everting balloon system, such as the inner catheter, can be withdrawn from the fallopian tube while leaving the fallopian tube occluding device in the fallopian tube. Once the everting balloon system is withdrawn from the fallopian tube, the fallopian tube occluding can be deployed (e.g., device anchors such as coils can be extended, or a resilient porous matrix can expand to friction fit the tube lumen). Once the fallopian tube occluding device is deployed, a central guidewire can be removed from the fallopian tube. The procedure can be repeated for the contralateral fallopian tube.

The everting balloon system can be used to access the bladder, ureters, kidneys, or combinations thereof. Devices, tools, instrumentation, endoscopes, drugs, therapeutic agents, sampling devices (brushes, biopsy, and aspiration mechanisms), or combinations thereof can be delivered through the inner catheter lumen to the target site.

### BRIEF DESCRIPTION OF THE DRAWINGS

Figures 1A through 1E are longitudinal cross-sectional views of the distal end of a variation of a method for using the everting balloon system.
Figure 2A illustrates a variation of the everting balloon system in a fully everted configuration.
Figure 2B is a cross-sectional view of a variation of the system handle.
Figure 3A illustrates a variation of the distal end of the everting balloon system with the dilating balloon in a less than fully inflated configuration.
Figure 3B illustrates a variation of the distal end of the everting balloon system with the dilating balloon in a fully inflated configuration.
Figure 4A illustrates a variation of the everting balloon system with a syringe in an attached, but not yet deployable configuration.
Figure 4B illustrates a variation of the everting balloon system with the syringe in an attached and deployable configuration.
Figure 4C illustrates a variation of the everting balloon system of figure 4B with the plunger driver shown in cut-away.
Figure 5A illustrates a length of a variation of the everting balloon system.
Figure 5B is a partial cross-sectional view of a variation of the system of figure 5A.
Figures 5C and 5D are variations of side and perspective views of a portion of cross-section A-A.
Figure 5E is an exploded view of a variation of a portion of the system handle and the drive gears.
Figure 5F is a close-up view of a variation of the system handle at the ratchet handle axle.
Figure 6A is a cross-sectional view of a variation of the system handle.
Figures 6B through 6D are side, top perspective and cross-sectional views, respectively, of a variation of the everting balloon system with the system handle of figure 6A.
Figures 7A and 7B are exploded and perspective views, respectively, of a variation of the everting balloon system.
Figure 8A is a cross-section view of a variation of the three-way connector and adjacent elements in a configuration to deliver media pressure to the outer catheter, for example to the everting balloon.
Figure 8B is a cross-section view of a variation of the three-way connector and adjacent elements in a configuration to deliver media pressure to the inner catheter, for example to the dilating balloon.
Figure 9 is an exploded view of a variation of a transfer catheter.
Figures 10A through 10C illustrate a variation of method for delivering material to a target site, such as reproductive material delivered to a uterine cavity.
Figures 11A through 11C illustrate a variation of a method for delivering material to a target site, such as reproductive material delivered to a uterine cavity.
Figure 12A is a longitudinal cross-sectional view of a variation of the distal end of the everting balloon system.
Figures 12B and 12C are close-up section A-A of the system of figure 12A with the outer catheter in configurations attached to the distal closure tip and detached from the distal closure tip, respectively.
Figure 13A is a cross-section view of a portion of a variation of the everting balloon system in a retracted configuration.
Figure 13B is a cross-section view of a portion of a variation of the everting balloon system in an extended or everted configuration.
Figure 13C illustrates a variation of a method for removing the everting balloon from a body lumen.
Figure 14A illustrates a variation of a portion of the everting balloon system with the plug inserted in the outer catheter distal tip.
Figure 14B illustrates the variation of the portion of the everting balloon system from figure 14A with the plug not inserted in the outer catheter distal tip.
Figures 15A illustrates a variation of a portion of the everting balloon system with the plug not inserted in the outer catheter distal tip and the everting balloon in a retracted configuration.
Figures 15B and 15C illustrate the variation of the portion of the everting balloon system of figure 15A with the plug inserted in the outer catheter distal tip and the everting balloon in a retracted configuration.
Figures 16A and 16B illustrate a variation of a portion of the everting balloon system with the everting balloon in everted and retracted configurations, respectively.
Figure 17 illustrates a variation of a method for pressurizing the everting balloon.
Figures 18A and 18B illustrate a variation of a method for pressurizing the everting balloon.

### DETAILED DESCRIPTION

An everting balloon system (also referred to as an everting catheter system) that can be used to traverse a vessel, such as the cervical canal is disclosed. The everting balloon system can be used to access the uterine cavity via the cervix. The cervical canal is a single lumen vessel that can stretch or dilate. The everting balloon system can have a control system that can be operated with one hand.

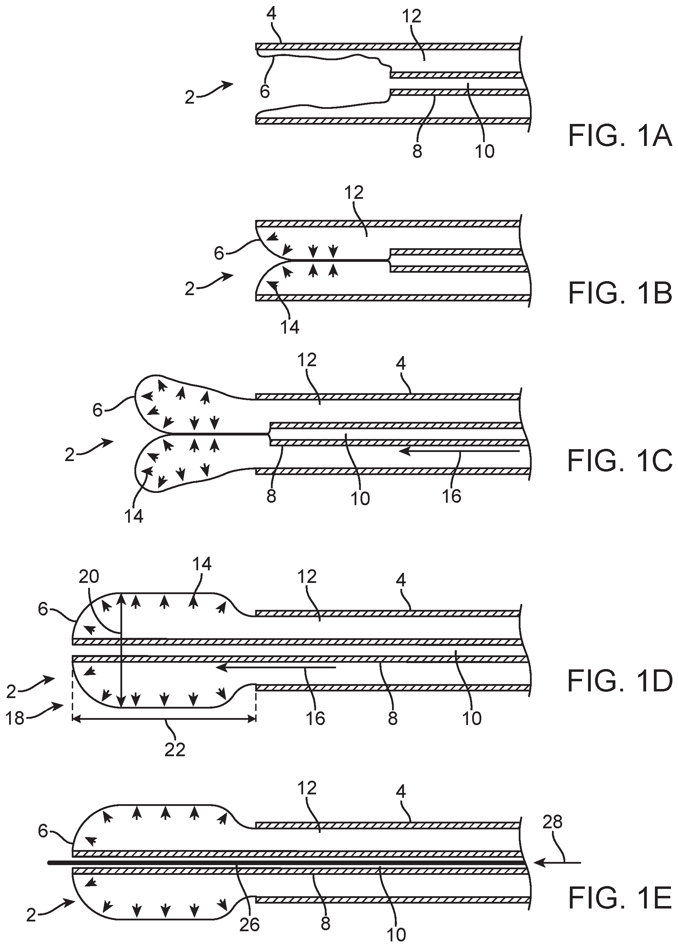

Figures 1A through 1E illustrate that an everting catheter system can have a radially outer catheter, a balloon membrane, and a radially inner catheter. The inner catheter can have an inner catheter lumen (e.g., a through-lumen). The distal end of the inner catheter lumen can be open or closed. The inner catheter can have the inner catheter lumen or be a solid rod or flexible mandrel. The everting balloon system can have a media volume. The media volume can be the contiguous open volume between the inner catheter and outer catheter that is proximal to the balloon membrane. A radially outer terminal perimeter of the balloon membrane can be attached to the distal terminal end of the outer catheter. A radially inner terminal perimeter of the balloon membrane can be attached to the distal terminal end of the inner catheter. The everting balloon system can be made without an inner catheter, for example with the balloon membrane extending proximally out of the working area to a control device (e.g., a pump).

Figure 1A illustrates that the everting catheter system can be in an unpressurized configuration. The media volume can be uninflated and unpressurized. The balloon membrane can be slack.

Figure 1B illustrates that that everting catheter system can be in a pressurized and uneverted configuration. A pressurization device, such as a pump, for example at the proximal end of the everting catheter system can be in fluid communication with the media volume. The pressurization device can deliver a fluid media, such as a pneumatic gas or hydraulic liquid media (e.g., saline, water, air, carbon dioxide, or combinations thereof), at a media pressure to the media volume. The media pressure in the everting balloon can be from about 2 to about 5 atmospheres of pressure when in the everted configuration and higher media pressures from about 5 atmospheres to 10 atmospheres are possible, for example, to provide greater everting capability for more difficult or stenotic passageways in the body.

The balloon membrane can inflate and be in tension. The balloon membrane can block the distal port of the inner catheter lumen.

Figure 1C illustrates that the everting catheter system can be in an inflated and partially everted configuration. The inner catheter can be translated distally, as shown by arrow, with respect to the outer catheter, and out of the outer catheter. The distal terminal end of the inner catheter can be proximal of the distal terminal end of the balloon membrane. The distal terminal end of the inner catheter can be proximal or terminal of the distal terminal end of the outer catheter. The balloon membrane can block the distal port of the inner catheter lumen or can be open allowing fluid communication between the inner catheter lumen and the target site.

Figure 1D illustrates that the everting catheter system can be in an inflated, fully everted, and fully distally extended configuration. The inner catheter can be translated distally, as shown by arrow, with respect to the outer catheter until the distal terminal end of the inner catheter is longitudinally beyond or co-terminal with the distal terminal end of the balloon membrane. The distal port of the inner catheter lumen can be unobstructedly accessible and in fluid communication with the target site.

In the fully inflated configuration, the balloon membrane can form an inflated everting balloon. The everting balloon can have a balloon outer diameter and balloon length in the inflated and fully everted configuration.

The balloon outer diameter can be from about 2 mm to about 20 mm, more narrowly from about 2 mm to about 7 mm, for example about 5 mm. The outer diameter can be constant or vary along the length of the everting balloon. For example, for use in the cervical canal, the most proximal portion of the everting balloon outer diameter could be configured with a smaller outer diameter than the remainder of the everting balloon membrane. As an example, the first proximal portion of the everting balloon can have a smaller balloon outer diameter such as from about 2 mm to 4mm for a length of from about 5 mm to about 10mm from the distal terminal end of the outer catheter, and the remainder of the length (e.g., from about 4 cm to about 7 cm along the everting balloon) of the everting balloon can have a balloon outer diameter from about 4 mm to about 7 mm. The outer diameter of the proximal end of the everting balloon can have a consistent balloon outer diameter, for example for delivery in the urethra, of from about 3 mm to about 6 mm, and the distal-most outer about 2 cm to about 3 cm of the everting balloon can have a balloon outer diameter from about 10 mm to about 20 mm, for example to create a seal with and anchor in the bladder.

The exterior surface of the balloon membrane can be configured with ridges, projections, bumps, grooves, and additional surface or mechanical features, or combinations thereof, for example for increased friction or holding power within the vessel.

The everting balloon length can be from about 2 cm to about 31 cm, more narrowly from about 2 cm to about 25 cm (e.g., for use in a male urethra), yet more narrowly from about 2 cm to about 7 cm, yet more narrowly from about 3 cm to about 6 cm, for example about 4 cm, about 7 cm, about 15 cm and about 30 cm.

Figure 1E illustrates that the everting catheter system can be in an inflated and partially or fully everted configuration. A tool, liquid, gas, or combinations thereof can be translated, as shown by the arrow, through the inner catheter lumen, out of the distal port of the inner catheter lumen and into the target site. The tool can be a biopsy tool, a scope, a sonogram probe, a plug, a cauterization tool, or combinations thereof. Suction can be applied from the proximal end of the inner catheter lumen, and to the target site, for example removing debris from the target site through the inner catheter lumen.

To retract and reposition or remove the balloon membrane, the inner catheter can be pulled proximally to pull the balloon membrane back within the outer catheter. The balloon membrane can be deflated or have media pressure reduced and the entire system can be withdrawn from the target site.

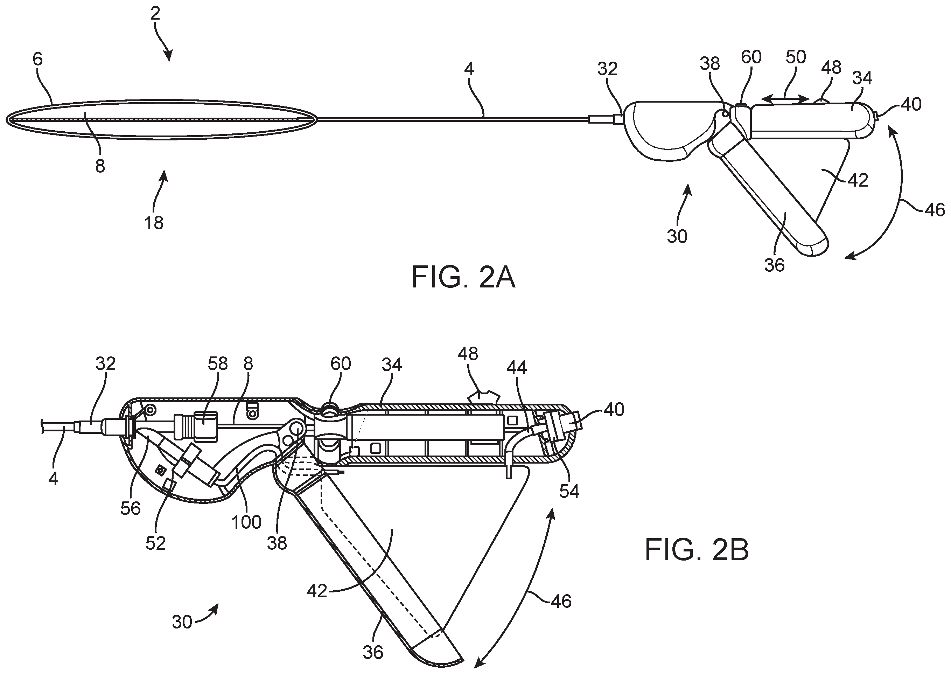

Figure 2 illustrates that the everting balloon system can have a system handle. The system handle can have a system handle connector. The system handle can be attached to the outer catheter and the inner catheter, for example at the system handle connector. The system handle connector can be removably attached to the outer catheter. For example, the outer and inner catheters and balloons can be detached from the system handle and replaced. The system handle can be sterilizable. Media (e.g., liquid or gas) delivered by the system handle can be filled into the system handle before attaching or replacing the catheters and balloons.

The system handle can have a rigid system handle case and a rigid pump lever rotatably attached to the system handle case at a pump lever axle.

The system handle can have an inlet port. The everting balloon system can have a pressurization source. The pressurization source can have a flexible liquid reservoir or fluid supply container or bag. The fluid bag can be filled with a hydraulic and/or pneumatic fluid.

The inlet port can be a female luer fitting and connection. The inlet port can be in fluid communication through an inlet-reservoir channel with the flexible reservoir. The liquid reservoir can be between the rigid pump lever and a rigid system handle case. The inlet port can extend out of the proximal end of the system handle case. The inlet port can be configured to attach to a liquid source (e.g., a hose, tube, or supplemental reservoir configured to deliver the liquid through the inlet port and to the liquid reservoir). The inlet port can have a proximal check valve or one-way valve configured to allow flow to the liquid reservoir and prevent backflow (e.g., proximal flow from the liquid reservoir and out the inlet port).

The liquid reservoir can be in one-way (e.g., via a check valve) or two-way fluid communication with the media volume.

When the liquid reservoir contains liquid, the pump lever can rotate away from the system handle case, as shown by pump lever rotation arrows, as the liquid reservoir inflates. The pump lever can be rotated toward the system handle case to compress the liquid reservoir, for example, forcing liquid from the liquid reservoir and into the media volume of the everting balloon.

The pump lever can provide a pumping (e.g., suction) action to supply aspiration to withdraw liquid from the media volume of the everting balloon. A spring within the lever can facilitate the pumping action of the lever to open the lever (not shown) for each compression.

The system handle can have an advancement slide. The advancement slide can be proximally and distally translatable, as shown by arrow, with respect to the system handle case. The advancement slide can be configured to translate the inner catheter with respect to the outer catheter. For example, pushing the advancement slide distally can push the inner catheter distally with respect to the outer catheter and evert the everting balloon. Pulling the advancement slide proximally can pull the inner catheter proximally with respect to the outer catheter and retract the everting balloon. The advancement slide can have gear wheels, ratchets with racks, and rotating advancement screws.

The advancement button can be an advancing ratchet or a roller wheel that is geared into or with the inner catheter to allow for translation of the inner catheter.

With one hand, the physician can advance the inner catheter, evert the everting balloon, traverse the cervical canal with the everting balloon, and access the uterine catheter through the inner catheter lumen.

The fluid reservoir can be pressurized prior to placement of the distal tip of the outer catheter at the cervix. The fluid reservoir can has a proximal check or one-way valve on the proximal portion of the handle. The proximal check valve is the connection point for the physician to pressurize the system. The distal portion of the fluid bag can be attached to a distal pressure check valve that can open when pressure from the fluid bag is at or above a distal check valve limit pressure, for example about 1 atmosphere of pressure from the liquid reservoir, and then deliver liquid and pressure from the liquid reservoir to fill and pressurize the media volume of the catheters and everting balloon. The distal pressure check valve can be a one-way valve allowing hydraulic or pneumatic fluid or media to go from the fluid reservoir to the media volume of the catheters and everting balloon. Higher and lower atmosphere pressure ratings from 1 atmosphere are also possible for the distal pressure check valve such as from about 0.5 atmospheres to about 2 atmospheres.

During pressurization of the fluid reservoir (e.g., by pumping with the pump lever or from the inlet port via the proximal check valve), pressures greater than a reservoir limit pressure (e.g., 1 atmosphere) of the distal pressure check valve can open the distal pressure check valve and allow fluid media to flow from the liquid reservoir into the media volume of the catheters and everting balloon. The pressurization in the media volume of the catheters and everting balloon can unroll and evert the everting balloon under hydraulic force. Excess media can remain in the fluid reservoir after the everting balloon fully everts.

The distal pressure valve can be connected to a three-way connector (e.g., Y-connector or T-connecter) that has a hemostasis valve, for example a Touhy-Borst valve. Thus the fluid reservoir can stage or hold additional potential hydraulic pressure to be stored in the system for the user (e.g., physician) to use as needed by rotating the pump lever without a change of hand position or the use of a second hand.

The inner catheter can extend through the three-way connector. The inner catheter can translate (i.e., advance and retract) through the three-way connector while maintaining a seal (i.e., without the media volume of the catheters or everting balloon losing pressure). The inner catheter (e.g., if a solid rod or mandrel) can be configured to withstand hydraulic pressures of up to about 5 atmospheres or up to about 10 atmospheres during the everting process and translational (e.g., advancement, retraction, tensile, compression, or combinations thereof) forces of up to about 2 pounds or up to about 5 pounds without deformation. As an example, during the everting process the inner catheter with an inner catheter lumen (e.g., a through lumen) could withstand media pressures, tensile and compressive forces, and rotational forces as the everting balloon membrane traverses curved or tortuous anatomy, to allow for the passage of an instrument, catheter, media, or materials within the through lumen. Movement of the advancement button on the handle moves the inner catheter within the three-way connector and through the outer catheter. The everting balloon can then evert and roll out of the outer catheter and traverse the target site (e.g., the cervical canal).

After accessing the target site, for example, the user can activate the pressure release control to release or reduce the pressure from the media volume thereby deflating or reducing the outer diameter of the everting balloon, and/or manually withdraw the everting balloon and inner catheter by retracting the advancement slide or pulling the system handle proximally, and therefore the remainder of the system.

Once the biological lumen to be traversed (e.g., the cervical canal, or urethra) is traversed by the everted balloon, the everting balloon system can increase the pressure in the everting balloon, for example increasing the diameter of the everting balloon, or while maintaining a constant diameter everting balloon (e.g., for a fiber-reinforced everting balloon or a balloon membrane constructed from a less distensible material). The pump lever can be compressed to increase pressure in the fluid reservoir builds and exits the distal pressure check valve. The proximal check valve can prevent or minimize the fluid media (e.g., pneumatic or hydraulic pressure) from leaking or bleeding in the proximal direction and out of the inlet port.

The user can rotate the pump lever, for example increasing the pressure in the fluid reservoir, the media volume, and the everting balloon. The balloon outer diameter can then increase, further pushing open the diameter of the biological lumen. For example, the everting balloon can dilate the cervix and cervical canal. Tools such as endoscopes, instruments, Hegars, other devices to increase the diameter of the cervix further, or combinations thereof, can then be inserted into the dilated cervical canal concurrent with the everting balloon system being located in the cervical canal or subsequent to the everting balloon system being withdrawn from the cervical canal.

The pump lever can deliver tactile feedback to the user indicating the pressure of the everting balloon. The everting balloon system can have a pressure gauge indicating the pressure in the media volume, such as in the liquid reservoir and/or the media volume in the catheters and everting balloon.

The system handle can have a pressure release control, such as a toggle lever or knob. The pressure release control can release fluid from the liquid reservoir and/or media volume of the catheters and everting balloon.

The pressure release control can be connected to the hemostasis valve. The hemostasis valve can have a seal or sealing gasket. The pressure release control can be configured to open and close the sealing gasket by rotating the sealing cap, or open a connection to a separate drainage tube (not shown) in fluid communication with the media volume.

The pressure release control can be on the handle positioned by the user's thumb position, distal to and collinear with the movement of the adjustment slide. The pressure release control can be operated by the same hand as the user is operating the adjustment slide and pump lever.

The user can perform the following operations of the everting balloon system with a single hand (e.g., without their other hand or another operator) without a change of hand position:
a. pressurize the liquid reservoir;
b. position or place the distal end of the everting balloon system at the patient's cervix;
c. control the everting balloon system position throughout use;
d. advance the inner catheter and balloon membrane;
e. increase the diameter of the everting balloon by pumping additional hydraulic pressure from the fluid reservoir;
f. retract the inner catheter and balloon membrane; and
g. activate the pressure release control to remove or release pressure from the everting catheter system.

Structurally, the buttons and actuators to enable these functions can be positioned on the handle to allow for the operator to manipulate these features without a change of hand position or requiring the use of the other hand. For instance, advancement and retraction of the inner catheter can be performed by a slide mechanism or gear wheels that are located on the upper side of the handle approximately 4 inches from the proximal end of the handle or handle grip. Levers and ratchet mechanisms can be located on the lower or underneath side of the handle at a distance of from about 2 inches to about 4 inches from the proximal end of the handle grip. Additional actuators can be placed on the lateral sides of the handle grip from about 3 inches to about 4 inches from the proximal end of the handle grip or on the upper or lower portions of the handle grip from about 3 inches to about 4 inches from the proximal end. The button and actuator position can be palpable for the operator without requiring visual confirmation, thereby allowing the user to maintain eye contact with the patient or visualization source such as an endoscopic monitor or ultrasound image.

During the use of the everting balloon system, the user can utilize their other hand for handling an ultrasonic probe, a tenaculum (e.g., if the cervix is difficult to access by anatomical reasons or is severely retroverted or anteverted), stabilizing the patient or other instruments, or combinations thereof.

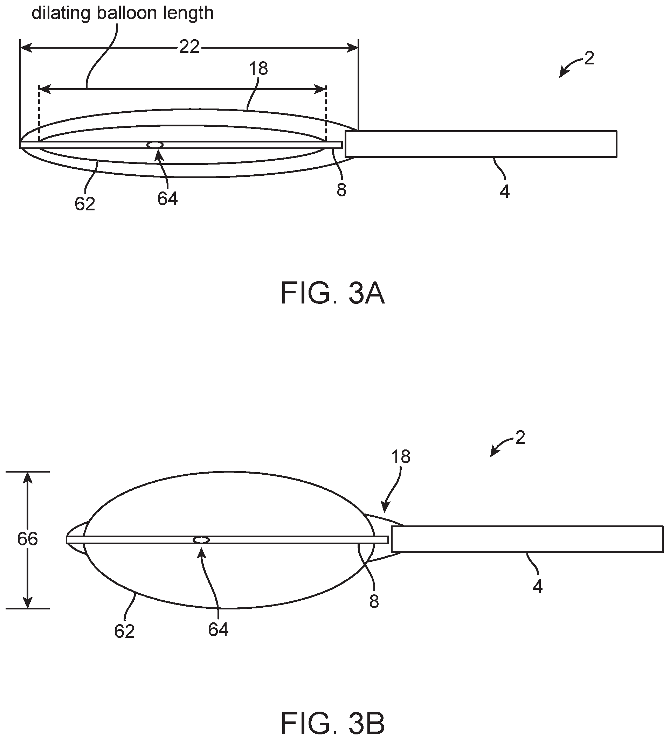

Figure 3A illustrates that the inner catheter can be attached to a dilating balloon or inner catheter balloon. The dilating balloon can be radially inside of the everting balloon. The distal end and the proximal end of the dilating balloon can be attached and sealed to the inner catheter. The inner catheter can have a dilating balloon port longitudinally within the dilating balloon. The dilating balloon port can be in fluid communication with a fluid pressure source at the proximal end of the everting balloon system, for example in or attached to the system handle. The dilating balloon can be inflated and deflated through the dilating balloon port.

The dilating balloon can be more, the same, or less compliant than the everting balloon. The everting balloon wall can be thicker, thinner, or the same thickness as the dilating balloon wall. The everting balloon can be made from one or more polymers including silicone, urethane, rubber, latex, polyethylene, polyolefin, irradiated polyolefin combined with ethylene vinyl acetate, co-polymers such as polyether block amide (PEBA, also known as Pebax), a fiber-reinforced polymer, PET, nylon, or combinations thereof. The dilating catheter can be made from any of the materials mentioned for the everting balloon.

The everting and/or dilating balloon membrane can have a thickness from about 0.001 in to about 0.004 in.

The everting and/or dilating balloon can be internally coated with a lubricious material such as silicone oil, mineral oil, other lubricant, or combinations thereof. The lubricous coating can reduce the friction within the balloon during eversion.

The exterior of the everting and/or dilating balloon can be smooth, for example the balloon can be made by tubing extrusion. The balloons can be blow molded. For example, the exterior surface of the balloon can have ridges or other surface protrusions, for example to increase friction or holding forces in the target body lumen (e.g., cervical channel or urethra). The outer diameter of the balloons can vary dimensionally. For instance, the most distal portion of the everting balloon can be manufactured with a larger outer diameter to accommodate larger vessel sizes or inflation that can extend into the bladder.

During use, the everting balloon can pull the inner catheter into the endocervical canal. When the everting balloon is deployed into the cervical channel, the dilating balloon can be positioned in the cervical channel.

Figure 3B illustrates that the dilating balloon can be inflated by delivering pressurized fluid through the dilating balloon inflation port. The dilating balloon can expand inside of the everting balloon. The dilating balloon can inflate to a dilating balloon diameter.

The dilating balloon can have a predetermined or molded size and shape. For example, the dilating balloon can have a dilating balloon diameter. For example, the maximum dilating balloon diameter or maximum everting balloon diameter can be from about 2 mm to about 12 30 mm, and for some applications, up to about 20 mm in diameter (e.g., for use in a cervix), and more narrowly from about 2 mm to about 10 mm (e.g., for use in a urethra), more narrowly from about 6 mm to about 12 mm, yet more narrowly from about 2 mm to about 7 mm (e.g., for use in a urethra), yet more narrowly from about 3 mm to about 4 mm (e.g., for use in a male urethra). The dilating balloon can inflate to a preset outer diameter. (The dilating balloon outer diameter can be equal to or less than the dilating diameter needed for the body lumen, such as the cervix.) The everting balloon can have a maximum everting balloon diameter equal to or less than the maximum dilating balloon diameter.

The dilating balloon can be inflated to the same or a higher pressure than the everting balloon. For example, the dilating balloon can have a dilating balloon pressure from about 4 atmospheres to about 12 atmospheres of pressure, and up to about 20 atmospheres of pressure, for example for disrupting a pathological stenosis or condition within a bodily lumen.

When the dilating balloon is inflated, the everting balloon can stretch due to the expanding dilating balloon to the dilating balloon diameter. The inflation media within the everting balloon can remain inside the balloon or be withdrawn before, during, and/or after inflation of the dilating balloon. Due to the frictional forces of the everting balloon membrane on the bodily lumen in the everted state, for example, the everting balloon membrane can serve to maintain the position of the dilating balloon during the dilation process without unintentional advancement or retraction of the system within the bodily lumen during the dilatation process.

The dilating balloon can inflate and tear or break the everting balloon as the everting balloon diameter expands beyond the strain limit for the everting balloon. The inflation media within the everting balloon can remain inside the balloon or be withdrawn before, during, and/or after inflation of the dilating balloon, for example exiting the everting balloon can exit when the everting balloon tears open.

The everting balloon can break or tear along an intentional line upon the inflation of the dilating catheter. For example, the everting balloon can be torn by a mechanical instrument on or within the outer catheter, a sharp implement on the proximal portion of the inner catheter that becomes active upon full eversion and inflation of the dilating balloon, and/or further advancement of the inner catheter that disengages the attachment or bond between the everting balloon and the inner catheter on the distal end of the inner catheter. The tearing or splitting of the everting balloon can be done be weakening the everting balloon with a mechanical indentation or seam on the balloon membrane that splits upon reaching a specific strain limit, such as along a helical line, lateral line, longitudinal line, or combinations thereof. The everting balloon membrane can be manufactured with increased longitudinal axial orientation of the molecular structure by tensioning or expanding the membrane along the longitudinal axis of the balloon during the balloon forming process which can promote a longitudinal break if the everting balloon membrane splits or tears. A radial tear in the everting balloon can be promoted by manufacturing the balloon membrane with greater radial orientation of the molecular structure by radially expanding or tensioning the balloon membrane during the balloon forming process.

The system handle can hold the inflation media to be delivered to and from the everting balloon and the dilating balloon. The inflation media can be in the liquid reservoir (e.g., the fluid bag or a syringe piston). The inflation media can be delivered, for example via valves, to the dilation balloon after the inflation and eversion of the everting balloon. The system handle can have gear wheels or a ratchet configured to advance the inner catheter. The outer catheter can extend about 25 cm distal to the system handle. The system handle and actuators can inflate the everting balloon and dilating balloon from control with one hand.

The dilating balloon can be positioned into and dilate the cervix.

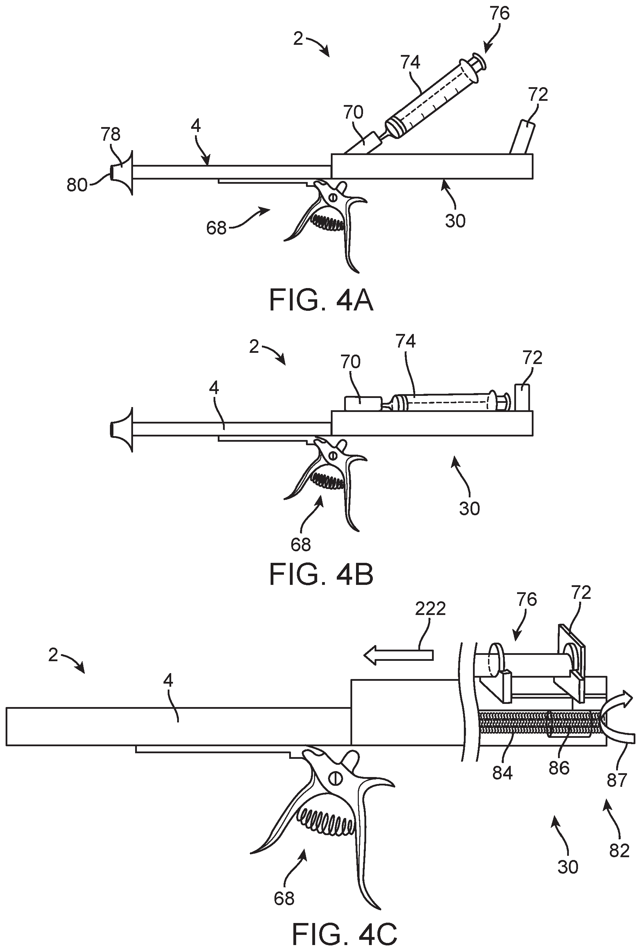

Figures 4A through 4C illustrate that the inner catheter can be in a fully retracted position inside of the outer catheter.

Figure 4A illustrates that the system handle can have a pump lever, such as a ratchet handle, a syringe connector, and a plunger drive plate. The ratchet handle can have a finger grip, trigger, lever, pump mechanism, or combinations thereof. The fluid reservoir can be a syringe. The syringe can have a volume from about 5 cc to about 20 cc, for example about 5 cc or about 20 cc. An open distal port of the syringe can be attached to and in fluid communication with the syringe connector. The syringe connector can have the distal pressure valve. The syringe connector can be rotatably attached to the system handle case. The syringe can have a plunger longitudinally translatable with the remainder of the syringe. The syringe can be filled with any media disclosed herein, such as saline, air, gas, or combinations thereof. The liquid reservoir can have two separate syringes, each attached to and in fluid communication with the same or different syringe connectors. For example, a first syringe can be in fluid communication with the everting balloon, and the second syringe can be in fluid communication with the dilation balloon.

The syringe can be locked to the syringe connector.

The outer catheter can have an outer catheter distal tip. The outer catheter distal tip can be, for example, an atraumatic tip such as an acorn tip or stop. The outer catheter distal tip can be configured to prevent insertion of the outer catheter too far into the target biological lumen (e.g., the endocervix).

The outer catheter distal tip can have an outer catheter distal port. The outer catheter distal port can be large enough to allow the inner catheter and balloons to pass through.

Figure 4B illustrates that the syringe connector and syringe can rotate, as shown by the arrow, so the longitudinal axis of the syringe can be parallel or collinear with the longitudinal axis of the outer catheter. The syringe connector can be angularly fixed with respect to the rest of the system handle. The plunger drive plate can be rotated and/or translated to contact or almost contact the proximal end of the syringe plunger.

Figure 4C illustrates that the system handle can have a plunger driver. The plunger driver can have a linear rack or plunger drive screw, plunger drive collar, and plunger drive plate. The ratchet handle can be squeezed to rotate the plunger drive screw, as shown by arrow, or linear rack. The plunger drive screw or linear rack can be configured to translate the plunger drive collar. For example, the plunger drive collar can have internal threads engaging with outer threads of the plunger drive screw. The plunger drive collar can be translatably fixed to the plunger drive plate. The plunger drive collar and plunger drive plate can translate distally with respect to the remainder of the syringe when the ratchet handle is squeezed. The plunger drive plate can be in contact with and press the plunger in a distal direction as shown by arrow.

The ratchet handle can have a ratchet to prevent reversing the direction of the plunger driver, for example to prevent proximal translation of the plunger. A release lever can be rotated or deployed to release the ratchet mechanism for disengagement of the assembly, withdrawal of the system, or redeployment. The ratchet handle can have no ratchet or a two-way ratchet, for example controlling the direction of the plunger driver, for example to allow proximal and distal translation of the plunger. The plunger drive plate can be fixed to or touching but unfixed to the plunger.

Squeezing the ratchet handle can depress the syringe plunger. Depressing the syringe plunger can force inflation media from the syringe to the media volume of the dilation and/or everting catheter, for example pressurizing the respective balloons.

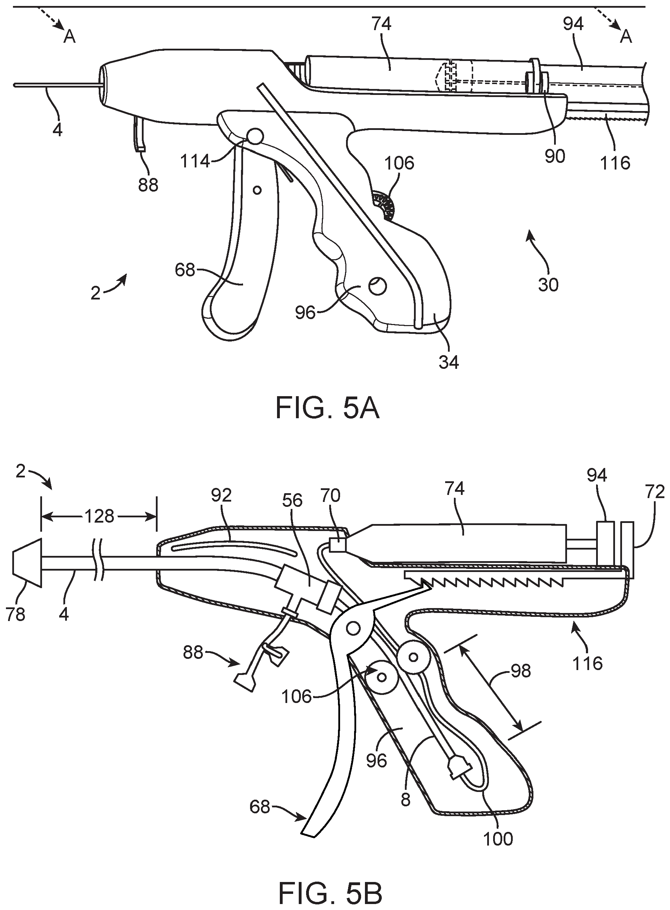

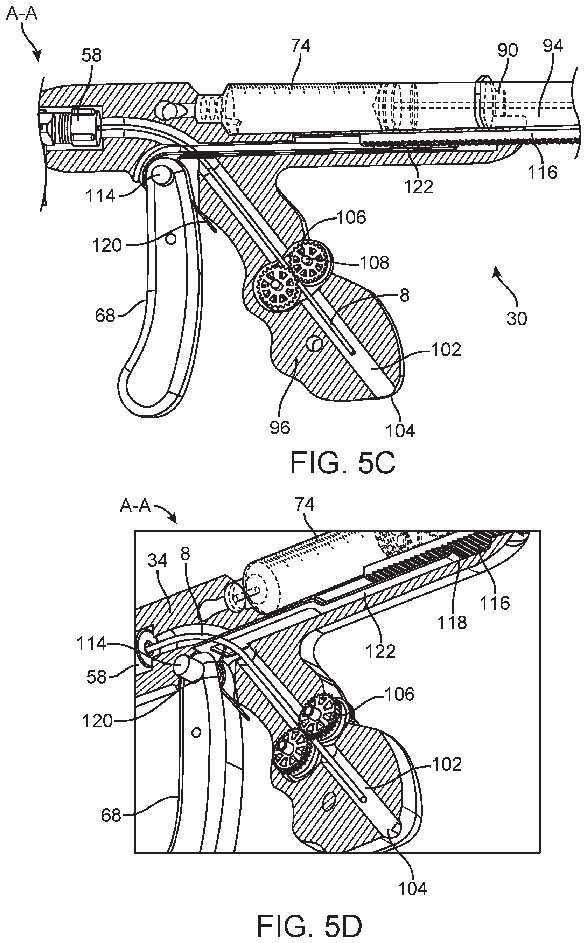

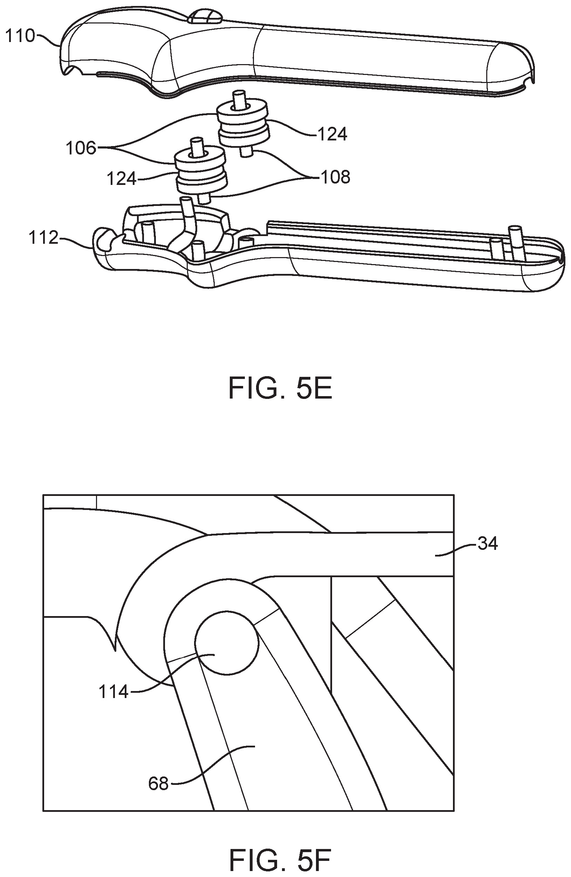

Figures 5A through 5F illustrates that the system handle can have a stop cock and check valve extending from the three-way connector. The stop cock and check valve can be in fluid communication with the media volume. The stop cock and check valve can be outside (as shown) or inside of the system handle case. The stop cock and check valve can be accessed to add media, remove media, or check the pressure of the media in the media volume.

The system handle can have one or more syringe detents. The syringe detents can removably attach to a portion of the syringe to prevent or minimize longitudinal translation of the syringe with respect to the system handle case. The syringe detent can be configured to allow the syringe to slide in and out of the detent transverse to the longitudinal axis of the syringe.

The system case handle can have a deflecting plate. The outer and/or inner catheters can press against the deflecting plate. The deflecting plate can alter or deflect the path of the outer and inner catheters towards the longitudinally axial direction of the target site. The deflecting plate can have a molded or formed groove, pins, plate, panel, or combinations thereof. The outer catheter can be manufactured with a preset curve to accommodate the curved path within the system handle case.

The system handle case can have a handle grip. The inner catheter can have a linear inner catheter grip length. The inner catheter grip length can be a length of the inner catheter in the uneverted state in the handle grip. The inner catheter grip length can be about 12 cm of inner catheter in the uneverted state, for example corresponding to an eversion length for the inner catheter grip length of about 6 cm (e.g., about 50% of the inner catheter grip length) of everted balloon membrane. Alternatively, the inner catheter can be configured to coil up on wheel, have telescoping segments, or have folding and unfolding segments, to reduce the amount of distance needed within a system handle case to accommodate the length of inner catheter in the uneverted state.

The system handle can have a reservoir-catheter channel, for example in fluid communication with the distal end of the syringe and the proximal end of the inner catheter. The reservoir-catheter channel can be a tube from the syringe connector to the inner catheter.

The system handle can have an access channel extending from an external surface of the system handle connector to an external surface of the system handle case. The access channel can proximally terminate at a proximal access port.

The inner catheter can extend through the access channel. One or more tools or fluids can be inserted through, and/or suction can be applied to, the proximal access port and access channel into and through or adjacent to the inner catheter.

The system handle can have one or more drive gears. The drive gears can be on one or opposite sides of the access channel. The drive gears can encroach or impinge into the access channel. The drive gears can be rotatably attached to the system handle case via drive gear axles. The drive gears can have teethed gear sections and drive gear grooves. The inner catheter can extend through the drive gear grooves. The drive gears can frictionally push and pull the inner catheter. One or more of the drive gears can extend and be exposed out of the system handle case. For example, the exposed drive gears can be rotated by pressing on the exposed drive gear with the user's palm or digit (e.g., thumb). The exposed drive gear can be interdigitally engaged with one or more non-exposed drive gears. Rotating a first one of the drive gears can rotate other drive gears interdigitally engaged with the first drive gear.

The system handle case can have a system handle case first lateral portion and a system handle case second lateral portion. The system handle can be made by attaching the system handle case first lateral portion to the system handle case second lateral portion. Each drive gear axle can be rotatably attached to the system handle case first lateral portion and the system handle case second lateral portion.

The pump lever axle can be a ratchet handle axle. The ratchet handle can rotate around the ratchet handle axle.

The system handle can have a plunger drive rack. The plunger drive rack can be fixed to the plunger drive plate. The plunger drive plate can extend perpendicularly from the proximal end of the plunger drive rack. A side of the plunger drive rack facing toward the plunger drive plate can have unidirectional or bidirectional drive teeth.

The system handle can have a ratchet handle spring compressed between the system handle case, and/or the ratchet handle, and/or a ratchet arm. The ratchet handle spring can reset the ratchet handle, for example by rotating the ratchet handle forward, after the ratchet handle has been squeezed.

The system handle can have the ratchet arm or actuating pawl. The ratchet arm can be mechanically attached to the ratchet handle, for example to the handle spring. The ratchet arm can be in a track limiting motion of the ratchet arm to translation in the longitudinal direction with respect to the syringe. The proximal terminal end of the ratchet arm can be curved in a u-shape. The terminal end of the ratchet arm can press against a ratchet tooth. The ratchet arm can be configured to pull the plunger drive rack distally when the ratchet handle is squeezed. The ratchet arm is configured to move proximally with respect to the plunger drive rack when the ratchet handle is returned to a reset position.

The system handle can have a locking pawl (not shown) can be spring-loaded between the system handle case and the plunger drive rack, for example, allowing distal translation of the plunger drive rack and preventing proximal translation of the plunger drive rack except when the locking pawl is manually released from the plunger drive rack by the release lever.

The outer catheter can have an outer catheter length, as shown in Figure 5B. The outer catheter length can be from about 4 cm to about 35 cm, more narrowly from about 10 cm to about 24 cm, for example about 17 cm.

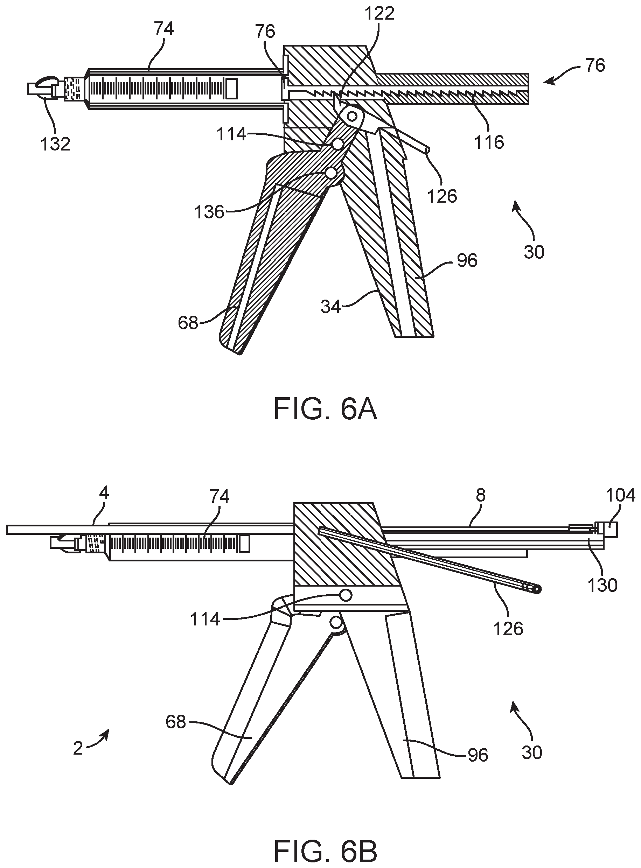

Figures 6A through 6D illustrate that the system handle can have an inner catheter drive tray translatably attached to the system handle case. A proximal length of the inner catheter can extend proximally from the system handle case. The proximal length of the inner catheter can be in, on, or adjacent to the inner catheter drive tray.

The syringe can have a syringe loading connector, such as a luer connector, at the terminal distal or proximal end of the syringe (e.g., the end further from the system handle case). A delivery tube or delivery device can be attached to the syringe loading connector and pressurized media can be delivered through the syringe loading connector into the syringe.

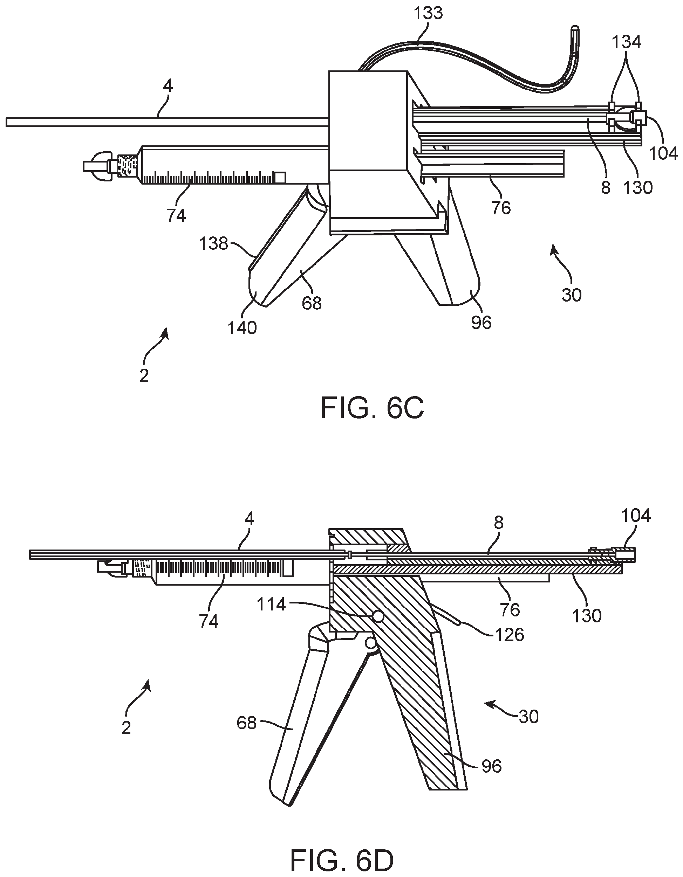

The delivery tube or delivery device can be disconnected from the syringe loading connector before deploying the everting balloon, as shown in figure 6C. The delivery tube can wrap inside the handle grip and connect the syringe and its pressurization media to the three-way connector and the hemostasis valve or inlet port for the dilation balloon.

The proximal terminal end of the inner catheter can be attached to the proximal access port. The proximal end of the inner catheter drive tray can have one or more access port detents. The access port detents can attach to the proximal access port. The access port detents can removably attach to a portion of the proximal access port to prevent or minimize longitudinal translation of the proximal access port with respect to the inner catheter drive tray. The access port detent can be configured to allow the proximal access port to slide in and out of the access port detents transverse to the longitudinal axis of the inner catheter drive tray.

The inner catheter drive tray can be translated along the longitudinal axis of the inner catheter drive tray to translate the inner catheter (e.g., advance the inner catheter into the target site).

The system handle case can have a fluid connection between the syringe and the outer catheter, as disclosed herein.

The ratchet arm can extend away from the drive rack to form a release lever, as shown in figure 6A. One or more other release levers can extend from other locations on the system handle, as shown in figures 6B and 6D. The release lever can be rotated to disengage the ratchet arm from the drive rack.

The ratchet handle can have a safety lock hole. A safety lock having a cable or rod can removably extend through the safety lock hole, for example to create an interference fit against the system handle case and prevent rotation of the ratchet handle, for example preventing unintentional or premature media delivery from the syringe.

The ratchet handle can be laterally split into a catheter sub-handle and a media sub-handle. The catheter sub-handle can be configured to control the advancement of the inner catheter drive tray. The media sub-handle can be configured to control the pressure of media delivery from the syringe. The catheter sub-handle can be attached to an inner catheter drive rack. The media sub-handle can be attached to a plunger drive rack.

The ratchet handle can control the syringe for applying media pressure to the everting balloon and dilating balloon, and independently control the translational movement of the inner catheter.

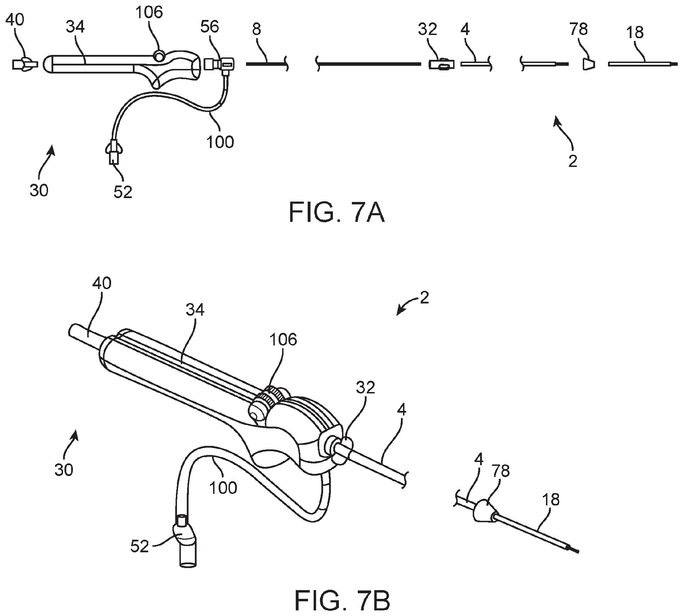

Figures 7A and 7B illustrate that the inlet port can have a female luer connector. The system handle connector can have a female luer connector. The outer catheter distal tip can have a soft rubber or polymerized acorn tip, for example, to assist in stabilizing the everting system at the opening of the bodily lumen or preventing unintentional advancement of the outer catheter within the bodily lumen.

The reservoir-catheter channel can extend from the three-way connector and out of the system handle case. The proximal terminal end of the reservoir-catheter channel can be attached to a female luer connector and/or the distal pressure valve. The distal pressure valve and/or female luer connector can be connected to the liquid reservoir (not shown).

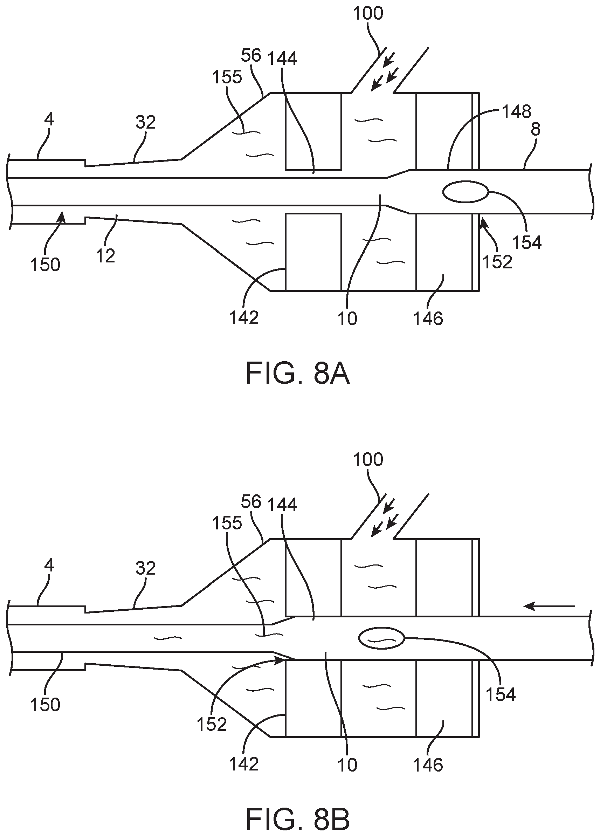

Figure 8A illustrates that the three-way connector can have a hemostasis valve. The three-way connector can have or be a Touhy-Borst Y-connector. The inner catheter can extend through the three-way connector.

The three-way connector can have a distal gasket between the reservoir-catheter channel and the system handle connector. The distal gasket can have a cylindrical distal gasket port extending through the radial middle of the distal gasket. The distal gasket port can have a distal gasket port diameter.

The three-way connector can have a proximal gasket proximal to the distal gasket. The proximal gasket can be between the reservoir-catheter channel and the proximal outlet through which the inner catheter proximally exits the three-way connector. The proximal gasket can be more, the same, or less compliant than the distal gasket. The proximal gasket can have a cylindrical proximal gasket port extending through the radial middle of the proximal gasket. The proximal gasket can have a proximal gasket port diameter.

The inner catheter can have an inner catheter small diameter length and an inner catheter large diameter length proximal to the inner catheter small diameter length. The inner catheter can have an inner catheter proximal inflation hole at the distal end of the inner catheter large diameter length. The inner catheter proximal inflation hole can be in fluid communication with the open distal end of the inner catheter lumen and/or the dilating balloon port.

Positive media pressure or flow can be delivered, as shown by arrows, through the reservoir catheter channel to the three-way connector. The inner catheter large diameter length can occlude, plug, and/or seal the proximal gasket port. The positive media pressure or flow can be delivered through the gap between the outer diameter of the inner catheter (e.g., along the inner catheter small diameter length) and the inner diameter of the distal gasket port and to the media volume between the outer catheter and the inner catheter, for example to the everting balloon.

Figure 8B illustrates that the inner catheter can be translated distally, as shown by arrow, at least until the inner catheter large diameter length moves into the distal gasket port. The inner catheter large diameter length can slide through the proximal gasket port. The inner catheter large diameter length can occlude, plug, and/or seal the distal gasket port and/or against the distal gasket. The media flow from the reservoir-catheter channel can be forced to flow into the inner catheter proximal inflation hole. The media can flow down the inner catheter lumen, for example to the dilating balloon.

An exemplary procedure for dilating the a body lumen such as the cervical canal can include:
1. The syringe can be loaded onto the system handle. The system handle can be a separate, reusable item in which the everting catheter and syringe filled with media can be attached to the remainder of the system before use. Alternatively, the system handle can come supplied to the end user preassembled with the remainder of the system and pre-filled, or combinations thereof.
2. The distal end of the everting balloon system can be placed at the exocervix.
3. The ratchet handle can be depressed. The first one to two clicks of the ratchet (i.e., as the locking pawl passes over ratchet teeth) can depress the syringe plunger and pressurize the everting balloon. The everting balloon can be pressurized to 4 to 6 atmospheres.
4. The ratchet handle can be depressed further (or released to rotationally reset and then depressed further). The next sets of clicks on the ratchet handle can indicate advancement the inner catheter. This can be accomplished by the ratchet mechanism rotating gear wheels on the inner catheter and/or translating a linear rack to advance the inner catheter.
5. The ratchet handle can be depressed further (or released to rotationally reset and then depressed further). The advancement of the inner catheter can continue until the everting balloon is fully deployed and everted. The dilation balloon can be positioned on the distal end of the inner catheter.
6. The ratchet handle can be depressed further. The next click of the ratchet can depressurize the everting balloon.
7. The ratchet handle can be depressed further. The next click of the ratchet can change the pressurization outlet of the syringe from the everting balloon to the dilation balloon. This can be accomplished, for example, by:
   a. rotating a valve with the ratchet mechanism,
   b. manually rotating the valve, and/or
   c. advancing the inner catheter to where the inner catheter proximal inflation hole or port is exposed to the inflation media, such as shown in figures 8A and 8B.
8. The ratchet handle can be depressed further. The next sets of clicks on the ratchet can indicate the inflation of the dilatation balloon.
9. The dilatation balloon may rupture the overlying everting balloon.
10. The amount of force in the biological lumen dilatation can be governed by a pressure relief valve or by the amount of volume of medial that can be placed within the dilatation balloon. The dilatation pressure can be monitored by a pressure gauge in or attached to the system handle case. The dilation balloon can dilate the cervix with from about 6 atmospheres to about 20 atmospheres. The dilation balloon can initially deliver about 10 atmospheres to about 12 atmospheres with a reduction in pressure as the cervix dilates and the dilatation process is completed. The system can deliver a known volume of media into the dilation balloon irrespective of quantifying or measuring the media pressure.
11. The dilatation process may be observed by ultrasound or radiographic imaging.
12. A pressure relief button on the system handle can be activated to remove or reduce dilatation pressure in the media volume in the inner catheter lumen.
13. The syringe plunger may be retracted to draw vacuum from the inner catheter lumen and dilation balloon, for example loosening the dilation balloon from the cervix, and/or deflating the dilation balloon, for example to facilitate removal of the everting balloon system from the cervix.
14. The everting balloon system can be re-pressurized, for example if additional dilatation force is desired in the cervix. For instance, if an additional stenosis in the cervix is visible, the dilatation balloon can be repositioned and inflated in the additional stenosis area.

The everting catheter system can access a bodily cavity (e.g., the uterine cavity or fallopian tubes) to deliver or introduce of tools (e.g., instruments), reproductive (e.g., embryos, in vitro fertilization (IVF) or insemination products, such as hormones) media or material, contrast media, dye, therapeutic agents, sclerosing agents to treat the endometrium, insufflation media, or combinations thereof to the cavity. For example, reproductive media can be delivered with a transfer catheter inserted through the inner catheter lumen to the uterine cavity.

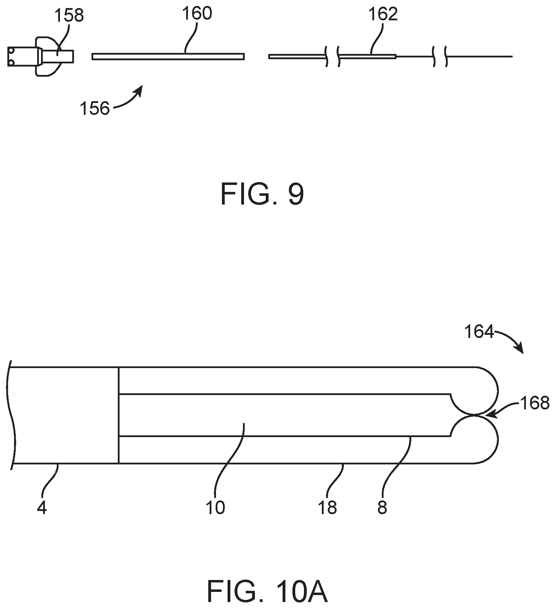

Figure 9 illustrates that a transfer catheter or insemination catheter can have a transfer connector, such as a female luer connector, a strain relief length, and a transfer tube. The transfer tube can hold the reproductive media. The transfer tube can have a proximal length having a proximal length diameter larger than a distal length diameter of a distal length of the transfer tube. A delivery force, for example a positive fluid pressure, can be delivered through the transfer connector and strain relief length to push the contents of the transfer tube into the target site.

The transfer catheter can attach to or inserted through the inlet port. The transfer tube can hold an embryo, for example for in vitro fertilization or IVF. The embryo transfer catheter can deliver embryos through the system and to the uterine cavity. The transfer catheter can hold spermatozoa and through the system and to the uterine cavity for intrauterine insemination procedures. The transfer catheter can hold and deliver other materials the deposition of drugs, therapeutic agents, instruments, endoscopes, cytology brushes, other catheters, or combinations thereof through the system and into the uterine cavity. The transfer catheter can be connected to a vacuum source for the aspiration of materials from the uterine cavity or other bodily cavities and lumens.

The transfer catheter and/or materials can be loaded in the inner catheter lumen prior to everting the everting balloon within the vessel or bodily cavity. For example in the case of delivery of reproductive material in the uterine cavity, the transfer catheter can be loaded with washed and prepared semen in the transfer tube and the transfer catheter can be placed in the inner catheter lumen.

A guidewire can be inserted through the transfer catheter and/or the remainder of the system, for example to direct the tube or system to the target site. The guidewire can be used for recanalization.

The inner catheter can be extended and the everting balloon can evert and unroll through the cervix and into the uterine cavity. Concurrently or subsequently, the transfer catheter can be advanced through the inner catheter lumen into the uterine cavity. Once fully everted or when the transfer catheter becomes extended or exposed from the inner catheter and beyond the everting balloon membrane, the reproductive material in the transfer catheter can be deposited by a syringe, squeeze bulb, piston, or other pressure system. A second delivery catheter, such as a second insemination, IVF, or drug delivery catheter can be concurrently inserted into the inlet port or a second inlet port. The second delivery catheter can be deployed to the target site concurrent with or subsequent to the transfer catheter.

The system handle can have a lead-in area. The lead-in area can, for example, be without steps, edges, bumps, or restrictions that may impede or contact the distal opening of the transfer catheter during passage, for example so that in the case of delivery of insemination material, the transfer catheter can be easily loaded into the system handle. An insemination syringe or pump can be attached to the proximal transfer connector to deliver pressure to the transfer tube, for example to expel the reproductive material once the distal port of the transfer catheter is positioned at the target site (e.g., after the everting balloon is fully deployed). The actuation of the insemination syringe or pump on a pre-loaded transfer catheter can be performed by the same hand that holds and operates the components of the everting catheter system.

In addition, the transfer catheter can be configured to be introduced into the proximal connector in the handle of the everting catheter system once the system is fully deployed.

The user can perform any or all of the following while using the everting balloon system, for example with a single hand:
a. pressurize the everting catheter system;
b. position the everting balloon system at the patient's cervix;
c. maintain the everting balloon system position throughout the procedure;
d. advance the inner catheter and everting balloon;
e. once extended beyond the everting balloon membrane or inner catheter, present the transfer catheter for deposition into the bodily cavity such as a uterine cavity
f. retract the inner catheter and everting balloon; and/or
g. activate (e.g., toggle) the pressure release lever to remove or release hydraulic or pneumatic pressure from the media volume.

Figures 10A through 10C illustrate that the distal end of the everting balloon can form a balloon check valve. The length of the everting balloon distal to the distal terminal end of the inner catheter can radially contract to form a tight orifice that can be the balloon check valve. The balloon check valve can be an openable barrier that can block or interrupt fluid communication between the inner catheter lumen and the target site.

The balloon membrane can balloon membrane have from about 1 mm to about 3 mm of overlapping wall at the balloon check valve closing off the inner catheter lumen. The strength or closing pressure of the balloon check valve can be modulated during use. For example the distance of overlap of balloon membrane can be increased or decreased by controlling the amount of excursion available for the inner catheter and everting balloon membrane.

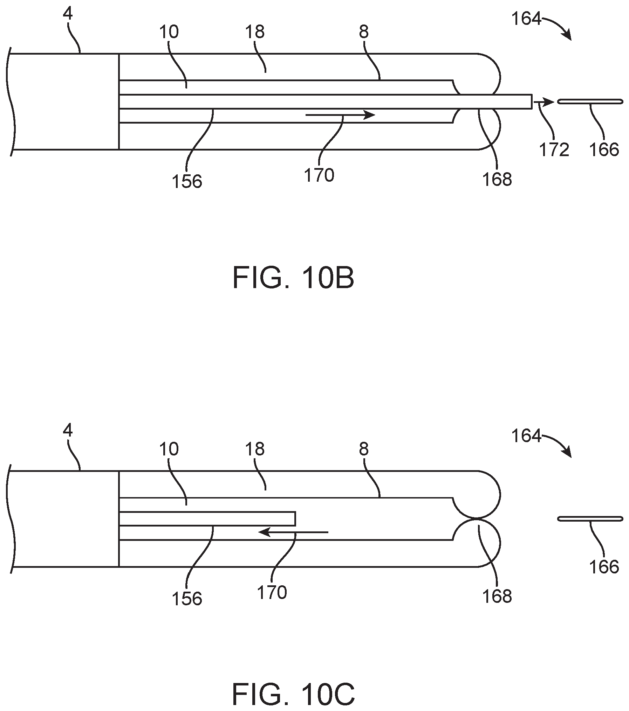

Figure 10B illustrates that the distal end of the transfer catheter can be advanced, as shown by arrow, through the inner catheter lumen, through the balloon check valve, and to the target site. The transfer catheter can penetrate or push open the balloon check valve when the transfer catheter moves through the balloon check valve. When the terminal distal end of the transfer catheter is distal of the balloon check valve and at the target site, the reproductive material loaded in the transfer catheter can be delivered through a distal port of the transfer catheter and into the target site, such as the uterine cavity.

Figure 10C illustrates that the transfer catheter can be retracted through the balloon check valve and the inner catheter lumen after the reproductive material is deposited at the target site. The balloon check valve can close as the transfer catheter is retracted through the balloon check valve. The balloon check valve can maintain a seal between the inner catheter lumen and the target site when the transfer catheter advances through, remains stationary within, and is retracted through the balloon check valve.

The reproductive material can be isolated from vacuum effect or the retraction of reproductive material from the target site as a result of the vacuum forces created by the withdrawal of the transfer catheter through the system once the deposition of reproductive material is completed. The balloon check valve can reduce or eliminate vacuum effect for embryo transfer.

The balloon check valve can be a tactile indicator for the physician when passing the transfer catheter through the everting balloon system. In transfer procedures, depending upon physician preference or patient anatomy, for example, the amount of insertion of the transfer catheter through the distal end of the everting system can vary from patient to patient. As the distal end of the transfer catheter passes through the balloon check valve, the resistance created by the balloon check valve can be felt by the physician on the proximal end of the transfer catheter. Depending upon the length of balloon chosen to act as a balloon check valve, the degree or amount of resistance can be modulated. In some procedural settings there may be a compromised ability to see the amount of insertion of the transfer catheter into the everting balloon, or physical depth indicia or markings on the proximal end of the transfer catheter. The compromised ability to see may be due to low light within the procedure room so that imaging and visualization of monitors can be enhanced. In addition, the physical relationship of the physician, embryologist, or other persons or equipment in the procedure room may compromise the ability to see easily the amount of insertion into the everting catheter. The tactile sensation of the resistance of the balloon check valve can create a palpable indicator that the transfer catheter is at the distal end of the everting balloon.

The everting balloon system can be used to access and seal the uterine cavity, for example, for the deposition of reproductive material for long duration intrauterine insemination.

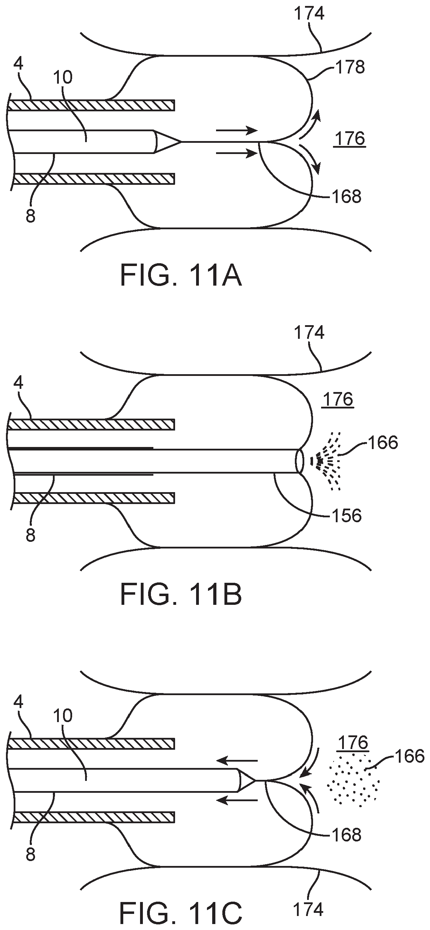

Figures 11A through 11C illustrate that the everting balloon membrane can create a seal within the cervical canal (e.g., against the cervical canal walls) as the everting balloon traverses the cervical canal. Figure 11A illustrates that the everting balloon membrane can unroll and advance along the cervical walls, as shown by arrows, as the balloon is pressurized and the inner catheter is distally advanced. The outer catheter can also seal against the cervical canal wall. For example, the outer catheter outer diameter can be equal to the everting balloon outer diameter.

Figure 11B illustrates that the transfer catheter can advance distally within the everting balloon and the inner catheter lumen. The transfer catheter can deposit the reproductive material (e.g., sperm) within the uterine cavity.

Figure 11C illustrates that the transfer catheter and/or the inner catheter can be retracted (e.g., from about 3 mm to about 10 mm) or inverted, as shown by arrows, to close the distal end of the inner catheter lumen, as shown by arrows, with respect to the uterine cavity. The distal opening of the balloon can close, for example due to the pressure within the everting balloon forcing the everting balloon to form the balloon check valve. The balloon check valve can seal the cervical canal and the uterine cavity from the inner catheter lumen. The reproductive materials can remain in the uterine cavity without being expelled through the cervix.

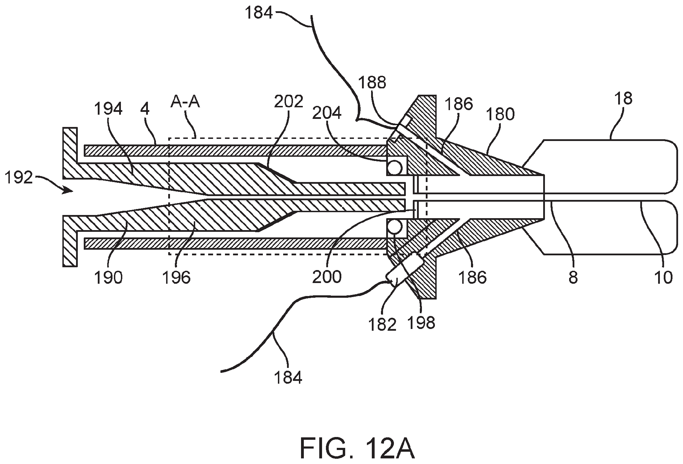

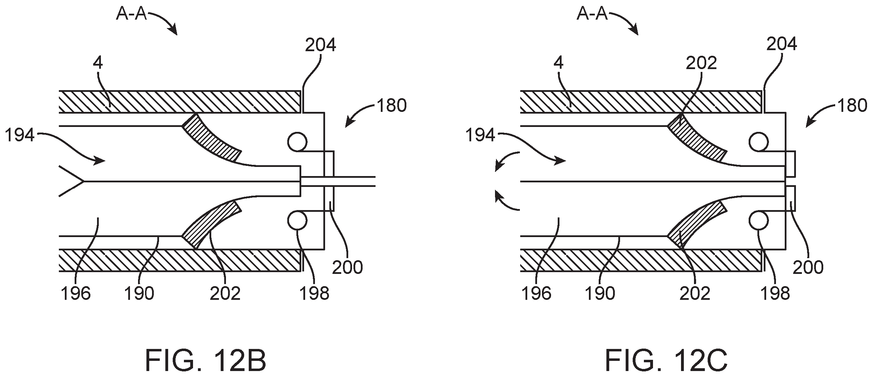

Figure 12 illustrates that the outer catheter and inner catheter can be configured to be de-coupled from the everting balloon membrane and a distal closure tip. The distal closure tip can be configured to connect to and seal off the everting balloon.

After delivery of the reproductive materials to the uterine cavity (i.e., insemination) is complete, the outer and inner catheters can be decoupled from the everting balloon and the distal closure tip. After the catheters are removed from the everting balloon, the everting balloon can remain inflated or pressurized. A one-way balloon closure check valve that can remain within the distal closure tip when the catheters are removed. The one-way balloon closure check valve can be connected to the balloon membrane with a conduit within the distal closure tip and can seal the everting balloon. A one-way check valve such as a duck bill valve within the distal closure tip can keep the reproductive material in the uterine cavity.

After the outer catheter and inner catheter are de-coupled from the balloon and removed from the cervix, the distal closure tip can remain in the cervical canal and/or the vagina. The distal closure tip can be made from biocompatible materials and a low durometer, soft and conformable silicone. The everting balloon can remain pressurized and sealing the cervical canal.

The distal closure tip can have a plug sealing the balloon and a pull string attached to the plug. (The pull string can be made from materials known for use for strings used commonly in tampons.) After a desired duration of time of keeping the cervical canal sealed without allowing the reproductive material (e.g., sperm) to exit the uterine cavity, and reproductive tract of the female, through the cervix, the everting balloon can be deflated and/or removed by pulling on the pull string to remove the plug, or pulling directly on the plug. The pull string can be connected to the plug. The plug can be connected to a proximal port of a pressurization release channel in fluid communication with the everting balloon. For example, the pull string is connected to the plug and the length string can continue further by being connected to the distal closure tip. Before, during, or after the everting balloon is deflated, the remaining elements can be removed from the cervix. The distal closure tip may or may not be removed until after the everting balloon membrane is deflated.

The pull string can be pulled by the physician or healthcare professional or alternatively, by the patient at home. The patient can be ambulatory while the everting balloon remains in the cervical canal. While sealed, the reproductive material can be contained within the uterine cavity without being expelled through the cervix by gravity, contractions, or movements by the patient.

The distal closure tip can have a pressurization release channel in fluid communication with the everting balloon and a pressurization release check valve. The pressurization release check valve can be a one-way check valve. The pressurization release check valve can be opened to depressurize and deflate the everting balloon. The deflated balloon can then be removed from the cervical channel by the patient or physician. For instance, the pressurization release check valve can have a duck bill valve that can allow air and fluid to travel into the distal tip and pressurize the everting balloon membrane. The everting balloon can be inflated or the internal pressure can be increased through the pressurization release check valve.

The pressurization release check valve can be attached to a pull string. For example, the pull string can be attached to the body of the duck bill valve. The user can pull the pull string, removing the duck bill valve from the distal closure tip, deflating the everting balloon, and then removing the distal closure tip and everting balloon.

Figures 12A, B, and C illustrate that the system can have an inner catheter plunger in the outer catheter. The proximal terminal end of the inner catheter plunger can have a plunger proximal connector. The plunger proximal connector can have a port or hole, for example configured to connect to a fluid delivery device to fill the everting balloon and/or plunger housing with pressurized media (e.g., saline, air). The everting balloon can also be pre-filled with media and fluidly sealed.

The inner catheter plunger can have a plunger first body and a plunger second body. The distal closure tip can have a plunger o-ring configured to form a seal around the inner catheter plunger where the inner catheter plunger enters the distal closure tip. The terminal proximal end of the inner catheter can be attached to inner catheter stop. The inner catheter stop can have a sealing gasket (e.g., the plunger o-ring and/or a different sealing element) exterior to and concentric with the inner catheter.

The diameter of the inner catheter plunger can decrease at a discreet slope along the length of the inner catheter plunger. The radially outer side of the distal end of the inner catheter plunger can have one or more mechanical decoupling detents, for example, on the discreet slope. The decoupling detents can extend radially beyond the radially outer surface of the remainder of the distal end of the inner catheter plunger.

The terminal proximal end of the distal closure tip can have one or more coupling connectors. The coupling connectors can have one or more flanges configured to releasably attach to the distal terminal end of the outer catheter.

The inner catheter plunger can be configured to distally advance the inner catheter. The inner catheter plunger can press against the inner catheter stop, translating and everting the inner catheter. After being fully distally advanced to a fully everted position, the inner catheter can be exposed to the target site and ready for the delivery of reproductive material to the target site. After delivery of the reproductive material to the target site, the inner catheter can be retracted, for example by retracting the inner catheter plunger, until a mechanical stop is reached and the plunger, plunger proximal connector, and outer catheter become disconnected from the inner catheter which remains within the distal closure tip. The sealing gasket can be on the exterior circumference of the inner catheter, for example, to prevent leaking of, and keep pressurization of the everting balloon pressurized. The outer catheter, containing the proximal connector and plunger, can be disconnected from a coupling with the distal closure tip by a number of possible manipulations including twisting, pulling, or pressing an actuator, or combinations thereof. The decoupling can be automatic when the distal end of the proximal connector mechanically engages and expands the flanges to release the coupling on the outer catheter.

Figures 12B illustrates that the inner catheter plunger can be in an attached configuration, not exerting radially outward pressure on the outer catheter.

Figure 12C illustrates that the proximal ends of the plunger first body and plunger second body can be rotated toward each other, as shown by arrows, causing a radially outward rotation of the distal ends of the plunger first and second bodies. The detents can press the outer catheter radially outward. The radial expansion of the outer catheter can expand the distal terminal end of the outer catheter. The outer catheter can then disengage from the coupling connector and the distal closure tip. The subsequent withdrawal of the outer catheter can remove the distal end of the proximal connector from the inner catheter. The inner catheter can remain in the distal closure tip.

After full deployment of the everting balloon membrane and deposition of reproductive material, the motion of the proximal connector in the proximal or retraction (e.g., reverse) direction to create a one-way balloon check valve can mechanically disengage the coupling connector (e.g., flanges) by use of ramps, detents, bumps, or steps that mechanically act on the flange, or the outer catheter housing itself, to then decouple the outer catheter, proximal connector and plunger from the distal closure tip.

The everting balloon can be pressurized through the pressurization release check valve within the distal closure tip and maintained by a plug with pull string attached. For example, the plug can be inserted into the pressurization release check valve. Any of the liquid reservoirs and respective pressurization systems can be used, such as a syringe, inflation device, and/or air or fluid pump.

The pressurization release check valve and the plug can be within the same component. For example, a releasable one-way valve such as a duckbill valve can be attached to a pull string. The pressurization release check valve and/or plug can be dislodged from the respective sealing areas, for example, providing reinflation for the everting balloon.

The everting balloon system can seal the cervical canal, for example, as follows:
a. The everting balloon can be pressurized by an inflation device or air/liquid pump before placing the device adjacent to a cervix.
b. The distal end of the everting balloon system can be placed at the patient's cervix.
c. The position of the everting balloon system can be maintained throughout the procedure (e.g., the device can operate via one-handed operation).
d. The inner catheter and everting balloon membrane can be distally advanced through the cervical canal.
e. The transfer catheter can advanced distally through and beyond the everting balloon or inner catheter.
f. The transfer catheter can deliver reproductive material into the uterine cavity or use a syringe to deposit the reproductive material through the proximal connector
g. The inner catheter and everting balloon can then be proximally retracted with respect to the target site.
h. The outer catheter and inner catheter can then be detached or decoupled from the distal closure tip and removed proximally from the distal closure tip. For example, the inflated distal tip can be left in the cervix and the everting balloon can remain pressurized.
i. Part or all of the everting balloon system can be left within the body for a predetermined duration of time
j. The pull string can be pulled to remove the plug and/or pressurization release check valves. The pull string can be connected to the plug to remove hydraulic pressure from the everting catheter system. The everting balloon can deflate.
k. The pull string can be attached to the everting balloon and/or distal closure tip and pulled to withdraw the deflated everting balloon and/or distal closure tip from the body.

The everting balloon system can be inserted into and occlude the urethra, for example to treat urinary incontinence in the male or female. The everting balloon can act as a urethral insert.

The everting balloon system can create a seal in a urethral channel or passage. Once positioned into or at the urethral opening, the user can press the surface of the outer catheter distal tip or everting balloon base against the urethral opening. The user can then activates the pressurization source and extrudes the everting balloon. The everting balloon system can self-propel along the urethral channel. The balloon material occupies the inner lumen of the urethra as it everts and thereby creates a sealing element. The everting balloon membrane can be filled with saline, air, or a combination of both.

The everting balloon can lock into place within the urethra. The everting balloon can deliver radial forces to the inner lumen of the urethra.

The everting balloon system can be compact and designed for self-insertion and removal by the patient. Bladder voiding can occur after removal of all or part of the system from the urethral passage or after a sealing mechanism in the system is inactivated or removed.

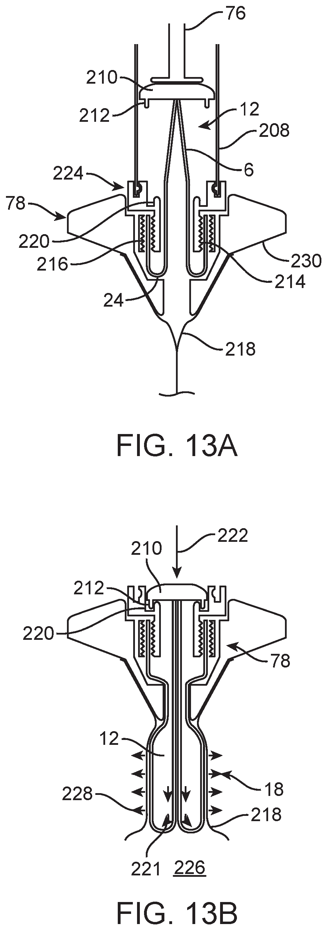

Figure 13A illustrates that the everting balloon system can have an outer catheter that is a syringe housing. A plunger within the syringe housing can be distally detachably connected to a media volume cap. The plunger can be proximally retracted, for example being ready for advancement. Part or all of the syringe housing, such as the portion within the everting balloon, can be filled with inflation media such as described herein. The syringe housing and/or plunger can be a reusable component of the everting balloon system that is attached to the remainder of the everting balloon system before deployment of the everting balloon by the user.

Cap lock pegs, collars, grooves, or a luer fitting can extend distally from or proximally into the radially outer portions of the distal and/or radial sides of the media volume cap.

The radially inner terminal end of the everting balloon membrane can be attached to the radially central portion of the cap lock. The cap lock can form a fluid-tight seal against the radial inside of the syringe housing.

The outer catheter distal tip can have a distal tip membrane interface. The distal tip membrane interface can have teeth or texturing (e.g., knurling, ridges, bumps) extending radially outwardly. The radially outer end of the balloon membrane can attach to the distal tip membrane interface. The outer catheter distal tip can have an everting balloon membrane collar. The everting membrane collar can have teeth or texturing (e.g., knurling, ridges, bumps) extending radially inwardly. The radially outer end of the balloon membrane can be squeezed between the everting membrane collar and the distal tip membrane interface to attach to the outer catheter distal tip.

The outer catheter distal tip can have a flange radially extending from the remainder of the outer catheter distal tip. The user can identify the urethral opening. The outer catheter distal tip can be positioned at the urethra opening and then translated and inserted into the urethra. As the outer catheter distal tip is translated into the urethra, the urethra can abut the flange, for example to stop translation of the outer catheter distal tip with respect to the urethra. The flange can be outside of the urethra in the male and outside the urethra in the vagina in the female. The outer catheter distal tip can be made from a low durometer, soft and conformable biocompatible material such as silicone.

The distal end of the syringe housing can detachably connect to the proximal end of the outer catheter distal tip at a fluid-tight tip connector. For example, the distal end of the syringe housing can detachably connect to the proximal end of the outer catheter distal tip at a circumferential snap connector or luer connector.

Cap lock ports, collars, grooves, or a luer fitting can extend distally from or proximally into the radially outer portions of the distal and/or radial sides of the media volume cap.

The outer catheter distal tip can act as the distal closure tip.

The syringe housing can be used as a handle to manipulate the position and orientation of the system.

Figure 13B illustrates that the plunger can be depressed or translated, as shown by arrow, toward the outer catheter distal tip, for example translating the media volume cap to press into the proximal end of the outer catheter distal tip.

The radially inner end of the balloon membrane and the inflation media in the media volume can be pressed distally, for example, causing the everting balloon to propel, unroll and evert, as shown by arrows, into the urethra. The cap lock peg can engage and attach the cap lock port. The cap lock peg attached to the cap lock port can form a fluid-tight seal. When the cap lock peg attached to the cap lock port, a palpable disengagement of the syringe housing can be felt by the user. The plunger and syringe housing can have thumb and finger rings, for example to separately control the plunger and the syringe housing with a single hand.

The syringe housing can be disengaged and detached from the inflation check valve and/or outer catheter distal tip at the tip connector and removed. The syringe housing can disengage from the outer catheter distal tip concurrently or subsequently to the cap lock peg attaching to the cap lock port.

The proximal end of the outer catheter distal tip and/or the distal end of the syringe can have a one-way inflation check valve, for example preventing or minimizing backflow of media into the syringe. The everting balloon can distend the urethra, as shown by arrows, and create a fluid-tight seal against the wall of the urethra. The everting balloon can automatically traverse the urethra, for example, without delivering a shear force to the urethra wall. The everted balloon can extend to the proximal terminal end of the urethra, into the bladder, or stop short of the proximal terminal end of the urethra.

Distal force can be delivered to the syringe housing, plunger, or outer catheter distal tip, for example to prevent dislodgement of the everting balloon during eversion, such as at the beginning of eversion before the everting balloon fixes to the urethra.

The everting balloon can be pressurized and can plug and seal the passageway of the urethra.

The everting balloon can seal throughout the urethral passageway by creating radial pressure throughout the urethra. Since the mechanism of action of the everting balloon is independent of urethral length, a sizing procedure is not needed. Avoiding multiple sizes simplifies physician fitting and does not require greater inventory.

The everting balloon can be soft and conformably anchored, for example, since the everting balloon fills space and thus exerted pressure evenly along the length of the everting balloon in the urethra. As the patient moves, bends, coughs, or provides pressure on the pelvic floor, the everting balloon can conform to the shape of the urethra, maintaining a seal until removed or deflated, for example by the patient.

The system can include an everting balloon that everts and expands immediately upon pressurization. This can be accomplished by having the most distal portion of the outer catheter distal tip constructed with exposed everting balloon membrane. In use, the most distal portion of the outer catheter distal tip can intubate the urethra with the exposed portion of the everting balloon membrane contacting the inner os of the urethra for a distance of 1 to 5 mm. Thus at pressurization, the everting balloon membrane can begin to lock into place and thereby reduce backing out. The remainder of the everting balloon can be maintained within the case unit up until the pressurization process is initiated. In this fashion, the invasive portion of the everting balloon that enters the urethra can be protected from contact from the user hands and other areas that can contaminate the balloon.

The everting balloon membrane can be housed completely within the distal outer catheter tip with the most distal portion of the distal outer catheter tip can be distensible and expand immediately upon pressurization of the everting balloon membrane. The distensible portion of the distal outer catheter tip can radially expand and contact the inner wall of urethra to provide locking forces.

A cylindrical open-ended tube can be used as an inserter to protect inadvertent contact of the everting balloon from vaginal side walls, for example, to prevent contamination of the everting balloon with vaginal flora or detritus during the insertion process.

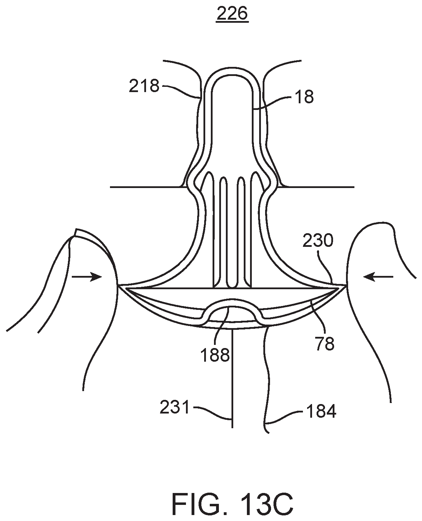

Figure 13C illustrates that the everting balloon can be deflated after insertion and deployment into the urethra. The everting balloon can be removed from the urethra and/or deflated, for example for bladder voiding (i.e., urinating). One or both of the flanges can be pinched or compressed toward each other by the user to deform the outer catheter distal tip and open the pressurization release check valve in the outer catheter distal tip. The inflation media can then exit the pressurization release check valve, deflating the everting balloon. The everting balloon and outer catheter distal tip can then be pulled out of the urethra and removed from the patient.

The outer catheter distal tip can have a pull string attached to the proximal end of the remainder of the outer catheter distal tip. The pull string can be pulled to remove the everting balloon and outer catheter distal tip (e.g., in the same fashion as the removal of a tampon).

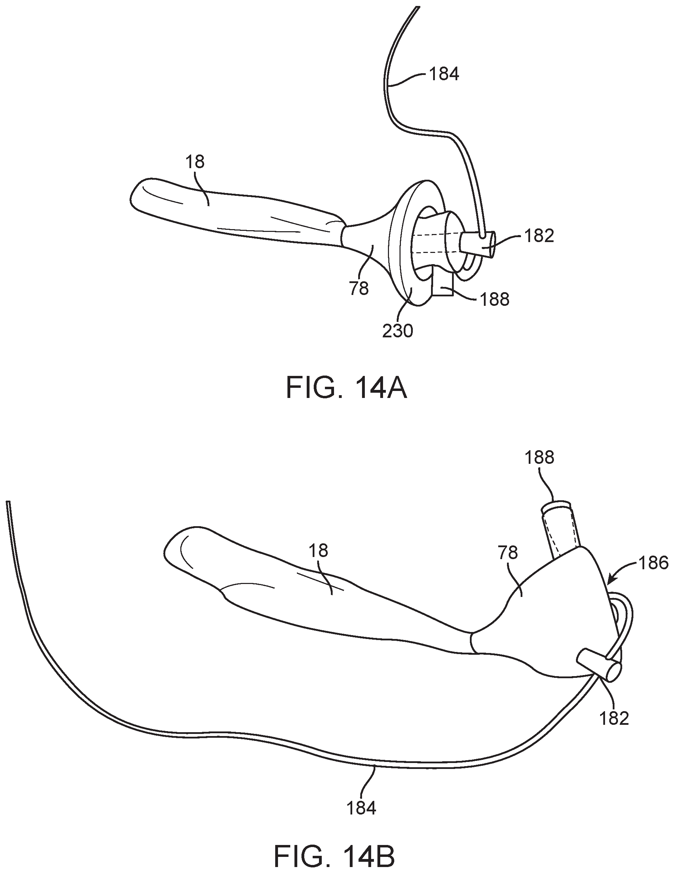

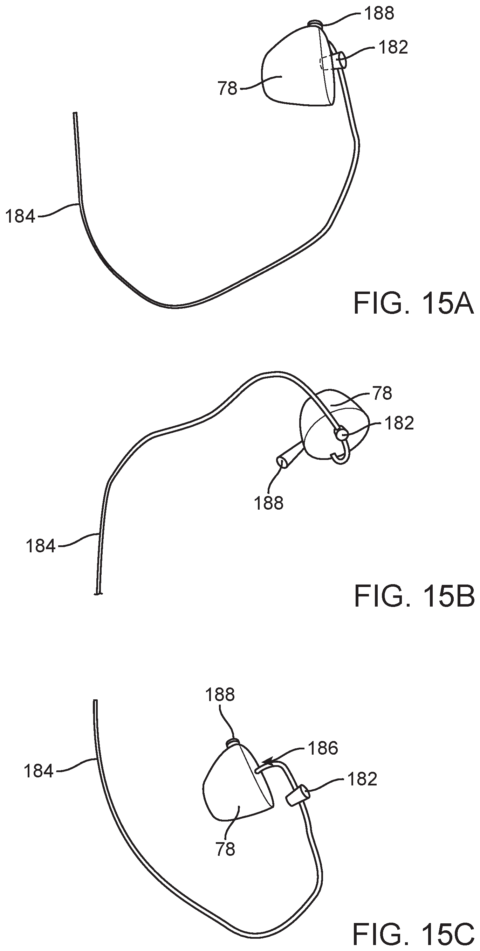

Figures 14A, 15A and 15B illustrate that the outer catheter distal tip can have a pressurization release check valve through which the media can enter to pressurize and inflate the everting balloon. The pressurization release check valve can be a one-way valve preventing or minimizing media flow out of the everting balloon to the surrounding environment. For example, the pressurization release check valve can have a duck bill or micro valve. The plug and external port of the respective pressurization release channel can be located radially central on the proximal side of the outer catheter distal tip.

Figures 14B and 15C illustrate that the pull string can be fixed to the body of the outer catheter distal port. The plug can be attached to the pull string. For example, the pull string can extend through the plug. For example, the plug can be glued to, welded to, clipped to, or over-molded onto the pull string. The pull string can be pulled to remove the plug from the external port of the pressurization release channel, and the media from the everting balloon can then be released to the surrounding environment. The everting balloon can then depressurize and deflate. The pull string can be pulled, removing the outer catheter distal tip and everting balloon from the urethra.

Once the positive lock mechanism is defeated by the pulled string, the everting balloon is deflated and the entire device can be removed and disposed.

Figures 14A and 14B illustrate that the distal terminal end of the outer catheter distal tip can have a distally protruding, pointed, acorn, nipple, or conical shape configuration.

Figures 15A through 15C illustrate that the distal terminal end of the outer catheter distal tip can have a smooth, rounded, concave shape that can minimize intubation into the urethra.

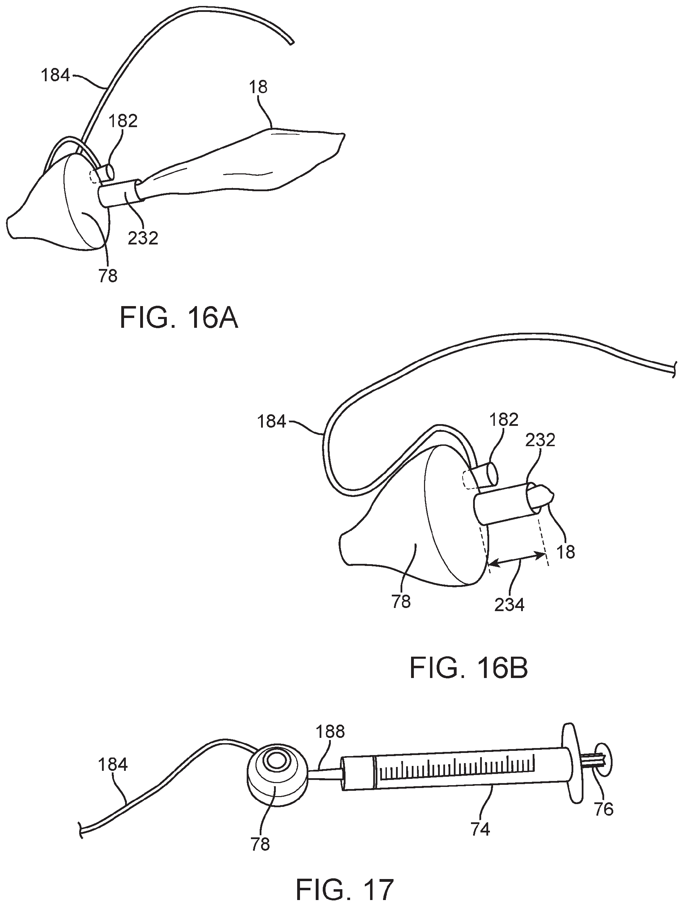

Figures 16A and 16B illustrate that the outer catheter distal tip can have a tip distal extension. The tip distal extension can extend distally from the remainder of the outer catheter distal tip. The tip distal extension can have a tip distal extension length. The tip distal extension length can be from about 1 mm to about 4mm. The tip distal extension can be cylindrical. The tip distal extension can be inserted into the body lumen, such as the urethra, before the everting balloon is everted.

Figure 17 illustrates that the inflation device can be a syringe. The syringe can be connected to the pressurization release check valve. The plunger can be depressed into the syringe, delivering media under pressure through the pressurization release check valve and into the everting balloon, for example, to inflate, expand, advance, propel, and evert the everting balloon.

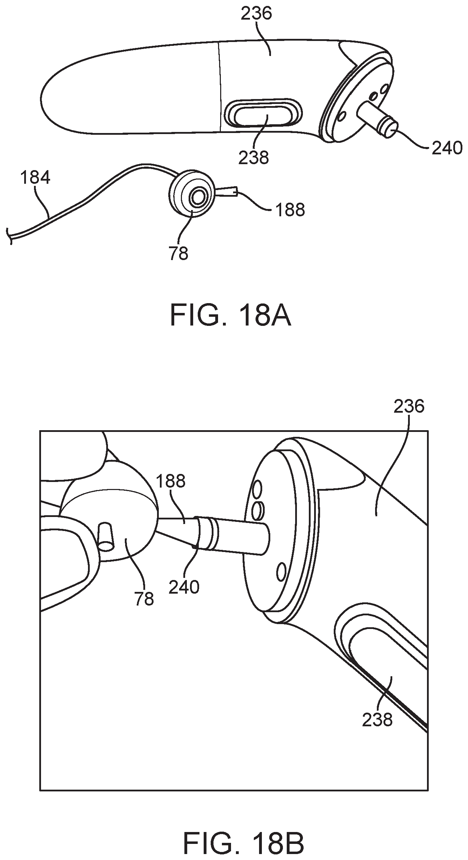

Figures 18A and 18B illustrate that the inflation device can be a small motorized air (or other gas or liquid) pump. The pump can have a pump power switch to control the pressure delivery from the pump. The pump power switch can be a push button or toggle. The pump power switch can control the flow rate and pressure of the media delivery from the pump. The pump can have a pump outlet configured to deliver pressurized media. The pump outlet can connect to the pressurization release check valve. The pump power switch can be turned to an on position. The motorized air pump can then deliver pressurized media through the pressurization release check valve and into the outer catheter distal tip. The pressurized media can, for example, inflate, expand, advance, propel, and evert the everting balloon.

The pump can deliver air or other media directly into the everting balloon. The air pump can be connected to the base of the everting balloon. The distal tip of the everting balloon can be placed at the urethral opening.

The pump can be configured to stall, a pressure relief valve, or other pressure check valve mechanism can open, or combinations thereof, if the pump outlet pressure exceeds a maximum pressure limit of the everting balloon.

After pressurization of the everting balloon, the pump can be disconnected from the pressurization release check valve, for example by the depression of a release button or can automatically be disengaged upon stalling the pump or by the activation of the pressure relief valve within the air pump. The increase of pressure within the everting balloon can automatically disconnect the air pump from the everting balloon and/or pressurization release check valve.

The outer catheter distal tip (i.e., the base of the everting balloon) can be small and made from a soft durometer material, with a shallow profile for comfortable wearing for the patient. The outer catheter distal tip can have a rounded, smooth, domed, or disk-like shape. The outer catheter distal tip can have a pillow-like feel. The outer catheter distal tip can be manufactured with two molded halves with an internal, circumferential attachment ring.

The pressurization release check valve can be engaged to the pump with an internal locking ring that can be disengaged either through a manual actuator or automatically as described above.

The everting balloon system can have a pressure indicator. The pressure indicator can indicate (e.g., via a light, buzzer, mechanical indicator such as a compliant dimple) if the desired pressure is not achieved, for example signifying a leak in the everting balloon system. The user can be alerted that the everting balloon system is not patent and should not be relied upon for effectiveness (e.g., urinary control).

The pump can be a reusable, small profile, battery operated, air pump. The pump can allow for insertion and control of the everting balloon and outer catheter distal tip at the urethra, inflation of the everting balloon, and can be disengaged from the everting balloon automatically or through a push button control or other actuator. As an example, the air pump can have a check valve for pressure relief at or below about 380mmHg (0.5 atm) or about 7psi (0.5 atm), for example, for inflation and sealing during exercise, coughing, and other pelvic floor movements by the user.

The air pump can be washed and replaced after a predetermined number of uses or applications before an internal battery is expended or replaced. The everting balloon can be mounted onto the distal end of the air pump, for example on or around the pump outlet.

The air pump can provide be operated with no manual manipulations by the user for inflation or locking air pressure. The air pump can be quiet and water-tight, for example to prevent leaking into the pump during cleaning.

The pump can be connected to the everting balloon or outer catheter distal tip by a section of tubing, for example to provide strain relief or more degrees of freedom in terms of insertion angle of the everting balloon. The air pump attached to the everting balloon can be delivered to the target site with one hand.

The inflation device can be or have a prefilled gas canister or ampule. The canister can be carbon dioxide (CO2) or nitrous oxide capsules.

The everting balloon can be pressurized with liquid (hydraulic) and/or gas (pneumatic) pressure. Liquid, such as water or saline, can be lubricous and incompressible. The liquid can deliver a higher traversing force for the everting balloon than a gas. Gas, such as carbon dioxide, nitrous oxide, nitrogen, or air, can be lighter than liquid.

The external surface of the everting balloon and/or outer catheter distal tip, for example the areas interfacing the urethra, can be coated or covered by conformable protrusions, gel or jelly, an adhesive-like covering, for example to secure to the target body lumen wall and prevent leaks, or combinations thereof. The coating on the external surface of the everting balloon can have anti-bacterial and/or anti-infection properties and/or agents to reduce the potential for infection. The coating can contain anesthetic and analgesic properties or agents (e.g. lidocaine) to reduce discomfort and/or other drugs, therapeutic agents, compounds, or combinations thereof. The external surface of the everting balloon and/or outer catheter distal tip, for example the areas interfacing the urethra, can be conformable and soft, for example to fill the target body lumen.

The everting balloon can be sterile before eversion. For example, all portions of the everting balloon can be contained inside the base when the system is positioned adjacent to the opening of the urethra. The distal outer catheter tip can be contained within an insertion tube during the insertion process.

Deployment of the everting balloon can be atraumatic. For example, the everting balloon can roll inside the body lumen (e.g., urethra) without any translational (i.e., shear) or frictional forces on the lumen wall.

The patient can keep and store additional everting balloon and outer catheter distal tips on their person.

As shown for example in figures 13A through 18B, the outer catheter distal tip does not need to attach to a catheter to be used. The outer catheter distal tip can be used by attaching to a pressurized media source (e.g., a catheter, syringe, pump, or combinations thereof), force delivery element (e.g., a plunger), having an on-board pressure source in the outer catheter distal tip, or combinations thereof. The outer catheter distal tip is also referred to as the base.

After the everting balloon system is pressurized and positioned in the cervix, the inner catheter can be distally advanced toward the target body cavity by hand or with a one-handed control system, such as the system handle. The system handle can allow the user to hold the entire catheter system and manipulate the pressurization, movement of components, and de-pressurization of the balloon membrane with one hand. The user can then utilize the other hand for the manipulation of instruments, controlling ultrasound, handling visualization techniques, depositing materials within the through-lumen by use of another syringe or delivery device mechanism, or combinations thereof.

Any elements described herein as singular can be pluralized (i.e., anything described as "one" can be more than one). Any species element of a genus element can have the characteristics or elements of any other species element of that genus. "Dilation" and "dilatation" are used interchangeably herein. The media delivered herein can be any of the fluids (e.g., liquid, gas, or combinations thereof) described herein. The patents and patent applications cited herein are all incorporated by reference herein in their entireties. Some elements may be absent from individual figures for reasons of illustrative clarity. The above-described configurations, elements or complete assemblies and methods and their elements for carrying out the disclosure, and variations of aspects of the disclosure can be combined and modified with each other in any combination. All devices, apparatuses, systems, and methods described herein can be used for medical (e.g., diagnostic, therapeutic or rehabilitative) or non-medical purposes.

As has become apparent from the above description, the present invention relates to the following aspects:
1. A method for accessing a cervical canal adjacent to a uterine cavity comprising:
   positioning an everting balloon system adjacent to the cervical canal, wherein the system comprises a catheter, a first balloon attached to the catheter, and a second balloon; and
   everting the first balloon, wherein everting comprises unrolling the first balloon inside of the cervical canal, and wherein everting comprises translating the second balloon into the cervical canal.
2. The method of aspect 1, further comprising inflating the second balloon in the cervical canal.
3. The method of aspect 2, wherein the inflating the second balloon comprises dilating the cervical canal.
4. The method of aspect 2, wherein the second balloon is less compliant than the first balloon, and wherein the inflating of the second balloon comprises tearing the first balloon.
5. The method of aspect 1, wherein the everting balloon system further comprises an elongated element, and wherein the first balloon is attached to the elongated element, and wherein the second balloon is attached to the elongated element.
6. The method of aspect 5, wherein the elongated element comprises a second balloon inflation port in fluid communication with the second balloon.
7. The method of aspect 5, wherein the elongated element has a lumen extending through the elongated element, and wherein the lumen is in fluid communication with the uterine cavity after the everting of the first balloon, and wherein the method further comprises translating a tool through the lumen to the uterine cavity.
8. The method of aspect 5, wherein the everting comprises at least one of pressurizing and inflating the first balloon.
9. A method for accessing a cervical canal adjacent to a uterine cavity comprising:
   positioning an everting balloon system adjacent to the cervical canal, wherein the system comprises a first catheter, a first balloon attached to the first catheter, and a second balloon; and
   everting the first balloon, wherein everting comprises unrolling the first balloon inside of the cervical canal; and
   inflating the second balloon in the cervical canal.
10. The method of aspect 9, wherein the inflating of the second balloon comprises dilating the cervical canal.
11. The method of aspect 10, wherein the second balloon is less compliant than the first balloon.
12. The method of aspect 10, wherein the inflating of the second balloon comprises tearing the first balloon.
13. The method of aspect 9, wherein the everting balloon system further comprises a second catheter, and wherein the first balloon is attached to the second catheter, and wherein the second balloon is attached to the second catheter.
14. The method of aspect 13, wherein the second catheter has a lumen extending through the second catheter, and wherein a distal terminal end of the lumen is closed.
15. A system for accessing a cervical canal adjacent to a uterine cavity comprising:
   a first catheter;
   a second catheter radially inside in the first catheter, wherein the second catheter is translatable with respect to the first catheter;
   a first balloon attached at a first end to the first catheter, and wherein the first balloon is attached at a second end to the second catheter; and
   a second balloon attached to the second catheter.
16. The system of aspect 15, wherein the second balloon is attached at a first end to the second catheter and at a second end to the second catheter, and wherein the second catheter has a port inside the second balloon.
17. The system of aspect 15, further comprising a connector having a first connector port, and a second connector port, wherein the second catheter extends through the first connector port and the second connector port.
18. The system of aspect 17, wherein the connector further has a third connector port, and wherein the connector and second catheter have a first configuration and a second configuration, and wherein in the first configuration the third connector port is in fluid communication with the volume between the first catheter and the second catheter, and wherein in the second configuration the third connector port is in fluid communication with a lumen in the second catheter.
19. The system of aspect 17, wherein the connector further comprises a first gasket having a first gasket port, and a second gasket having a second gasket port, and wherein the second catheter extends through the first gasket port and the second gasket port.
20. The system of aspect 17, wherein the second catheter has a first length having a first diameter, and a second length having a second diameter, and wherein the first diameter is larger than the second diameter.
21. The system of aspect 20, wherein the second catheter has a port on the lateral side of the second catheter in the second length, and wherein the port is in fluid communication with the second balloon.
22. A system for accessing a cervical canal adjacent to a uterine cavity comprising:
   a first catheter;
   a second catheter radially inside in the first catheter;
   an everting balloon;
   a dilation balloon; and
   a handle comprising a first control, a second control, and a third control, wherein the first control is configured to translate the second catheter, and wherein the second control is configured to inflate the everting balloon, and wherein the third control is configured to inflate the dilation balloon.
23. The system of aspect 22, wherein the everting balloon is attached at a first end to the first catheter and at a second end to the second catheter; and wherein the dilation balloon is inside of the everting balloon when the dilation balloon is in a deflated configuration.
24. The system of aspect 22, wherein the second catheter is translatable with respect to the first catheter.
25. A method for delivering matter into a uterine cavity comprising:
   positioning an everting balloon system adjacent to a cervical canal, wherein the everting balloon device comprises a first catheter and an everting balloon attached to the first catheter, and wherein the first catheter has a catheter lumen and a distal port at the distal end of the catheter lumen;
   everting the everting balloon in the cervical canal, wherein the everting comprises pulling the first catheter distally through the cervical canal, and wherein the everting comprises inflating the balloon distal to the first catheter, wherein the inflating comprises fluidly sealing the distal port with respect to the uterine cavity.
26. The method of aspect 25, wherein the sealing comprises forming a check valve comprising at least a portion of the balloon distal to the first catheter.
27. The method of aspect 26, further comprising delivering a tool through the catheter lumen and through the check valve to the uterine cavity.
28. The method of aspect 26, further comprising delivering a reproductive material through the catheter lumen and through the check valve.
29. The method of aspect 25, further comprising detaching the everting balloon from the first catheter while the everting balloon is in the cervical canal.
30. The method of aspect 25, further comprising detaching the everting balloon from the first catheter while the everting balloon is inflated.
31. The method of aspect 25, further comprising detaching the everting balloon from the first catheter, and maintaining inflation of the everting balloon after the detaching.
32. A method for delivering matter into a uterine cavity comprising:
   everting a balloon in a cervical canal, wherein the balloon is attached to a first catheter, and wherein everting comprises pulling the first catheter distally through the cervical canal;
   transporting a reproductive material through the first catheter into the uterine cavity;
   occluding the cervical channel with the balloon after the transporting of the reproductive material, wherein the occluding comprises occluding with the everting balloon.
33. The method of aspect 32, further comprising removing the everting balloon from the cervical channel.
34. The method of aspect 32, further comprising inflating the balloon distal to the first catheter
35. The method of aspect 32, further comprising fluidly sealing a distal port of the first catheter with respect to the uterine cavity.
36. The method of aspect 32, wherein the occluding comprises sealing the reproductive material from moving into the cervical canal,
37. The method of aspect 32, wherein the sealing comprises forming a check valve comprising at least a portion of the balloon distal to the first catheter.
38. A system for delivering matter into the reproductive tract of a female comprising:
   a first catheter having a lumen and a distal lumen port, wherein the first catheter has a retracted configuration and an extended configuration;
   an everting balloon attached to the first catheter, wherein at least a length of the everting balloon extends past the distal end of the first catheter when the first catheter is in the extended configuration;
   a second catheter slidably located in the first catheter; and
   a reproductive material in the second catheter.
39. The system of aspect 38, wherein the length of the everting balloon that extends past the distal end of the first catheter comprises a check valve when the first catheter is in the extended configuration.
40. The system of aspect 38, further comprising a distal closure tip, wherein the distal closure tip is attached to the everting balloon.
41. The system of aspect 40, wherein the distal closure tip is configured to detach the everting balloon from the first catheter and the second catheter.
42. The system of aspect 41, wherein the distal closure tip comprises a pressure releasing element.
43. The system of aspect 38, further comprising a third catheter radially outside of the first catheter.
44. The system of aspect 43, wherein the everting balloon is attached at a first end of the everting balloon to the third catheter, and wherein the everting balloon is attached at a second end of the everting balloon to the first catheter.
45. The system of aspect 38, where the reproductive tract of the female comprises a fallopian tube.
46. The system of aspect 45, where the second catheter is configured to deliver a tubal occlusion device.
47. The system of aspect 38, further comprising a handle configured to control the control eversion of the everting balloon.
48. The system of aspect 47, wherein the handle is configured to control translation of the second catheter.
49. A method for treating a biological lumen comprising:
   positioning a balloon adjacent to an opening of the biological lumen;
   delivering a pressurized media into the balloon with an inflation device, wherein the delivering comprises everting the balloon into the lumen, and wherein the everting comprises occluding the lumen;
   detaching the inflation device from the balloon; and
   removing the balloon from the lumen after the detaching of the inflation device.
50. The method of aspect 49, further comprising depressurizing the balloon, wherein the balloon is attached to a base, and wherein the depressurizing comprises removing a plug from the base.
51. The method of aspect 49, further comprising depressurizing the balloon, wherein the balloon is attached to a base comprising a valve, and wherein the depressurizing comprises opening the valve.
52. The method of aspect 49, further comprising depressurizing the balloon, wherein the balloon is attached to a base comprising a string, and wherein the removing the depressurizing comprises pulling the string
53. The method of aspect 49, wherein the balloon is attached to a base comprising a string, and wherein the removing the balloon comprises pulling the string.
54. The method of aspect 49, wherein the balloon has a lumen extending through the length of the balloon.
55. The method of aspect 54, further comprising occluding the lumen with a plug.
56. The method of aspect 49, wherein the biological lumen comprises a urethra.
57. The method of aspect 49, wherein the delivering comprises inflating the balloon.
58. A method for treating a biological lumen comprising:
   positioning a balloon adjacent to an opening of the biological lumen;
   delivering a pressurized media into the balloon, wherein the delivering comprises everting the balloon into the lumen, and wherein the everting comprises occluding the lumen, wherein the everting comprises pressurizing and inflating; and
   wherein the biological lumen comprises a urethra.
59. A system for treating a biological lumen comprising:
   an everting balloon;
   a base attached to the everting balloon, the base having a port;
   a plug attached to the port.
60. The system of aspect 59, further comprising an inflation device detachably connected to the base.
61. The system of aspect 60, wherein the inflation device comprises a catheter.
62. The system of aspect 60, wherein the inflation device comprises a syringe.
63. The system of aspect 59, wherein the inflation device comprises an electrical pump.
64. The system of aspect 59, wherein the base has a flange, and wherein the base is compliant, and wherein the base has a valve, and wherein the valve is configured to open when force is exerted on the flange.
65. The system of aspect 59, further comprising a check valve.
66. The system of aspect 65, wherein the check valve is a one-way valve configured to allow flow in the direction of the everting balloon.
67. The system of aspect 59, further comprising a string attached to the plug.
68. The system of aspect 67, wherein the string is attached to the base.

## Claims

1. A system for delivering matter into the reproductive tract of a female comprising:
a first catheter having a lumen and a distal lumen port;
a second catheter adapted to be slidably inserted in the first catheter;
a third catheter radially outside of the first catheter;
wherein the first catheter can be translated distally within the third catheter and has a retracted configuration and an extended configuration in relation to the third catheter;
an everting balloon attached at a first end thereof to the third catheter and attached at a second end thereof to the first catheter,
an inflation device for pressurizing said everting balloon,
wherein a radially outer terminal perimeter of the everting balloon is attached to a distal terminal end of the third catheter, and a radially inner terminal perimeter of the everting balloon is attached to a distal terminal end of the first catheter, wherein at least a length of the everting balloon extends past the distal end of the first catheter when the first catheter is in the extended configuration, wherein the distal end of the length of the everting balloon that extends past the distal end of the first catheter forms, when pressurized by said inflation device, a check valve when the first catheter is in the extended configuration and the distal lumen port of the first catheter is distal of the distal terminal end of the third catheter, wherein the check valve is at an orifice at the distal end of the length of the everting balloon.

2. The system of claim 1, further comprising a handle configured to control the eversion of the everting balloon.

3. The system of claim 2, wherein the handle is configured to control translation of the second catheter.

4. The system of claim 1, wherein the second catheter is configured to deliver a tubal occlusion device.

5. The system of claim 4, wherein the tubal occlusion device is a fallopian tube occlusion device.

6. The system of claim 1, wherein the second catheter is a transfer catheter for delivery of reproductive material to the uterine cavity.

7. The system of claim 1, wherein the second catheter can penetrate or push open the check valve when the second catheter moves through the check valve.

8. The system of claim 1, wherein the check valve formed by the everting balloon can maintain a seal between the first catheter lumen and a target site of the reproductive tract when the second catheter is retracted through the check valve.

9. The system of claim 1, further comprising a first catheter atraumatic tip.

10. The system of claim 9, wherein the first catheter atraumatic tip has an acorn shape.

11. The system of claim 1, wherein the everting balloon has a length between 2 cm and 31 cm.

12. The system of claim 1, wherein the everting balloon has an outer diameter between 2 mm and 20 mm.

13. The system of claim 1, further comprising a second catheter distal tip, wherein the second catheter distal tip is extendible through the check valve.

14. The system of claim 1, wherein a second catheter distal tip is configured to open the check valve.

15. The system of claim 1, further comprising a hormone in the second catheter.
